(19) **Europäisches Patentamt**
**European Patent Office**
**Office européen des brevets**

(11) **EP 4 385 507 A1**

(12) **EUROPEAN PATENT APPLICATION**

(43) Date of publication:
**19.06.2024 Bulletin 2024/25**

(21) Application number: **22213139.3**

(22) Date of filing: **13.12.2022**

(51) International Patent Classification (IPC):
**A61K 31/337** (2006.01)   **A61K 31/427** (2006.01)
**A61P 35/00** (2006.01)

(52) Cooperative Patent Classification (CPC):
(C-Sets available)
**A61K 31/337; A61K 31/427; A61K 45/06;**
**A61P 35/00** (Cont.)

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Stichting Het Nederlands Kanker**
**Instituut-**
**Antoni van Leeuwenhoek Ziekenhuis**
**1066 CX Amsterdam (NL)**

(72) Inventors:
- **BEIJNEN, Jacob Hendrik**
  **Amsterdam (NL)**
- **van EIJK, Maarten**
  **Amsterdam (NL)**
- **HUITEMA, Alwin Dagmar Redmar**
  **Amsterdam (NL)**
- **LOOS, Nancy Hélène Catharina**
  **Amsterdam (NL)**
- **SCHINKEL, Alfred Hermanus**
  **Amsterdam (NL)**

(74) Representative: **Simmons & Simmons LLP**
**Claude Debussylaan 247**
**1082 MC Amsterdam (NL)**

(54) **COMBINATION TREATMENT FOR SOLID TUMORS USING CABAZITAXEL AND A CYP3A INHIBITOR**

(57) The current invention relates to a pharmaceutical composition comprising an effective amount of cabazitaxel, or a CYP3A inhibitor, for use in a combination treatment in the treatment of cancer in a patient. The combination treatment comprises administering the pharmaceutical composition and the CYP3A inhibitor, and the dose of the CYP3A inhibitor is equivalent to 40 to 120 mg ritonavir. The current invention also relates to method and kit for the combination treatment thereof.

Figure 30

**EP 4 385 507 A1**

**(Cont. next page)**

(52) Cooperative Patent Classification (CPC): (Cont.)

C-Sets
**A61K 31/337, A61K 2300/00;
A61K 31/427, A61K 2300/00**

**Description**

The invention relates to chemotherapy of tumors using taxanes, in particular cabazitaxel. More in particular it relates to a combination treatment of cabazitaxel and a CYP3A inhibitor that achieves efficacious exposures of cabazitaxel whilst maintaining acceptable toxicity (of both the cabazitaxel and the CYP3A inhibitor).

**Background**

Treatments of cancers involve a wide range of treatments, including for example surgery, radiation therapy, chemotherapy, immunotherapy and cell therapy. Often, cancer treatments include a combination of different modes of treatments, comprising combinations of different therapeutic agents. Taxanes such as docetaxel, cabazitaxel and paclitaxel are often used as the cytotoxic agent for first line and/or second line chemotherapy in the treatment of various cancers, such as breast cancer, non-small lung cancer, and metastatic castration-resistant prostate cancer.

Cabazitaxel is also a semisynthetic taxane. The only difference between the molecular structures of docetaxel and cabazitaxel is the replacement of two hydroxyl groups by methoxy groups. Its main mode of action is understood to involve interference with microtubule assembly and disassembly, resulting in inhibiting mitotic cell division. While having the potential to benefit patients, improving life expectancy and quality of life, the use of cabazitaxel comes along with significant side effects. Typical side effects include i.a. neutropenia, a high risk of infections, thrombocytopenia, anemia, alopecia, fluid retention, diarrhoea, nail toxicity, peripheral sensory neurotoxicity and infusion related reactions. Cabazitaxel (at a dose of 25 mg/m2 of body surface) is nowadays administered as a one hour intravenous infusion every three weeks. The recommended mode of use involves a restricted number of cycles, usually 4-6 cycles, of cabazitaxel, to limit side effects. In addition, standard premedication with high dose dexamethasone is needed every cycle. Intravenous infusion not only is demanding for patients as it usually requires hospital visits, but also leads to potential secondary side effects associated with intravenous therapy.

There is therefore a need to improve bioavailability of cabazitaxel, in order to manage side effects whilst maintaining therapeutic efficacies of the treatment.

**Summary of the invention**

The current inventors sought to provide for improved means and methods for utilizing cabazitaxel in the treatment of cancer. Surprisingly, it was found that when using a low dosage CYP3A inhibitor together with a pharmaceutical composition of cabazitaxel, an effective cabazitaxel treatment could be obtained having less of the side effects, including primary side effects from both therapeutic agents as well as, optionally, secondary side effects from administration.

According to a first aspect, the present invention provides a pharmaceutical composition comprising an effective amount of cabazitaxel for use in a combination treatment in the treatment of cancer in a patient, wherein the combination treatment further comprises a CYP3A inhibitor, and wherein the dose of the CYP3A inhibitor is equivalent to 40 to 120 mg ritonavir. According to a second aspect, the present invention provides a CYP3A inhibitor for use in a combination treatment in the treatment of cancer in a patient, wherein the combination treatment further comprises a pharmaceutical composition comprising an effective amount of cabazitaxel, and wherein the dose of the CYP3A inhibitor is equivalent to 40 to 120 mg ritonavir. According to a third aspect, the present invention provides a method for the treatment of a cancer in a patient. The method comprises: providing a pharmaceutical composition comprising an effective amount of cabazitaxel; and providing a CYP3A inhibitor, wherein the dose of the CYP3A inhibitor is equivalent to 40 to 120 mg ritonavir. By providing methods and uses combining cabazitaxel with a low dose CYP3A inhibitor, the inventors have established improved means and methods for the treatment of cancer, said methods and means providing for an improved safety profile of cabazitaxel as compared with the standard of care treatment for cabazitaxel, while at the same time allowing to exert control to obtain efficacious anti-tumor levels of cabazitaxel exposure. Peak concentration of cabazitaxel as measured in plasma (which can also be measured in serum, or whole blood) can be better controlled, thereby allowing for extended periods of administration. The level of exposure of cabazitaxel achieved with the combination treatment of the present invention is comparable or at least comparable to a standard of care treatment for cabazitaxel.

Alternatively, CYP3A activity of subjects and/or cabazitaxel plasma levels of patients that are to undergo the combination treatment and/or are in combination treatment, in accordance with the invention, can be determined in order to adjust cabazitaxel and/or CYP3A inhibitor dosage to control and monitor plasma levels of cabazitaxel that are sufficient to maintain exposure levels in the tumor tissue at least comparable to a standard of care treatment for cabazitaxel.

In particular, the present invention provides, as a fourth aspect, a method for the treatment of cancer in a patient. The method comprises the step of: determining the activity of CYP3A in the patient; optionally, comparing the activity of CYP3A to a reference level; and determining a CYP3A inhibitor dose, within a range equivalent to 40 to 120 mg ritonavir, based on the activity level of CYP3A determined in the patient.

According to a fifth aspect, the present invention further provides a kit comprising a pharmaceutical composition comprising an effective amount of cabazitaxel and a CYP3A inhibitor.

**Figures**

In Figure 1, plots are shown presenting pharmacokinetic parameters of cabazitaxel in male wild-type, Cyp3a-/-, and Cyp3aXAV mice over 24 hours after oral administration of 10 mg/kg cabazitaxel in combination with vehicle or 25 mg/kg ritonavir (n = 6).

In Figure 2 and Figure 3 plots are shown indicating tissue distribution and brain distribution of cabazitaxel in male wild-type, Cyp3a-/-, and Cyp3aXAV mice 24 hours after oral administration of 10 mg/kg cabazitaxel in combination with vehicle or 25 mg/kg ritonavir (n = 6).

In Figure 4 plots are shown presenting various pharmacokinetic parameters of cabazitaxel in male wild-type and Cyp3aXAV mice over 24 hours after oral administration of 10 mg/kg cabazitaxel in combination with vehicle, 1 mg/kg ritonavir or 10 mg/kg ritonavir (n = 6).

In Figure 5 plots are shown presenting pharmacokinetic parameters of cabazitaxel and its active metabolites in male wild-type, Cyp3a-/-, and Cyp3aXAV mice over 24 hours after oral administration of 10 mg/kg cabazitaxel in combination with vehicle or 25 mg/kg ritonavir (n = 6).

In Figure 6 plots are shown presenting pharmacokinetic parameters of cabazitaxel and its active metabolites in male wild-type and Cyp3aXAV mice over 24 hours after oral administration of 10 mg/kg cabazitaxel in combination with vehicle, 1 mg/kg ritonavir or 10 mg/kg ritonavir (n = 6).

In Figure 7 plots are shown presenting pharmacokinetic parameters of ritonavir in male wild-type, Cyp3a-/-, and Cyp3aXAV mice over 24 hours after oral administration of 25 mg/kg ritonavir (n = 6).

In Figure 8 plots are shown presenting pharmacokinetic parameters of ritonavir in male wild-type, and Cyp3aXAV mice over 24 hours after oral administration of 1 mg/kg or 10 mg/kg ritonavir (n = 6).

In Figure 9 plots are shown indicating tissue distribution of cabazitaxel in male wild-type, Cyp3a-/-, and Cyp3aXAV mice 24 hours after oral administration of 10 mg/kg cabazitaxel in combination with vehicle or 25 mg/kg ritonavir (n = 6).

In Figure 10 plots are shown presenting percentages of dose of cabazitaxel recovered in small intestinal contents (SIC) in male wild-type, Cyp3a-/- and Cyp3aXAV mice 24 h after oral administration of 10 mg/kg cabazitaxel in combination with vehicle or 25 mg/kg ritonavir (n = 6).

In Figure 11 plots are shown presenting pharmacokinetic parameters of cabazitaxel in male wild-type and Cyp3aXAV mice over 24 hours after oral administration of 10 mg/kg cabazitaxel in combination with vehicle, 1 mg/kg ritonavir, 10 mg/kg ritonavir, or 25 mg/kg (n = 6).

In Figure 12 plots are shown presenting pharmacodynamically active metabolite-to-cabazitaxel ratios in male wild-type, Cyp3a-/-, and Cyp3aXAV mice over 24 hours after oral administration of 10 mg/kg cabazitaxel in combination with vehicle or 25 mg/kg ritonavir (n = 6).

In Figure 13 and figure 14 plots are shown presenting tissue distribution of the metabolite DM1 in male wild-type, Cyp3a-/-, and Cyp3aXAV mice 24 hours after oral administration of 10 mg/kg cabazitaxel in combination with vehicle or 25 mg/kg ritonavir (n = 6).

In Figure 15 and figure 16 plots are shown presenting tissue distribution of the metabolite DM2 in male wild-type, Cyp3a-/-, and Cyp3aXAV mice 24 hours after oral administration of 10 mg/kg cabazitaxel in combination with vehicle or 25 mg/kg ritonavir (n = 6).

In Figure 17 and figure 18 plots are shown presenting tissue distribution of the metabolite docetaxel in male wild-type, Cyp3a-/-, and Cyp3aXAV mice 24 hours after oral administration of 10 mg/kg cabazitaxel in combination with vehicle or 25 mg/kg ritonavir (n = 6).

In Figure 19 plots are shown presenting pharmacodynamically active metabolite-to-cabazitaxel ratios in male wild-type and Cyp3aXAV mice over 24 hours after oral administration of 10 mg/kg cabazitaxel in combination with vehicle, 1 mg/kg ritonavir or 10 mg/kg ritonavir (n = 6).

In Figure 20 plots are shown presenting tissue distribution of ritonavir in male wild-type, Cyp3a-/-, and Cyp3aXAV mice 24 hours after oral administration of 25 mg/kg ritonavir (n = 6).

In Figure 21 plots are shown presenting pharmacokinetic parameters of ritonavir in male wild-type, and Cyp3aXAV mice over 24 hours after oral administration of 1 mg/kg, 10 mg/kg, or 25 mg/kg ritonavir (n = 6).

In Figure 22 plots are shown presenting pharmacokinetic parameters of cabazitaxel combined with its active metabolites DM1, DM2, and docetaxel in male wild-type, Cyp3a-/-, and Cyp3aXAV mice over 24 hours after oral administration of 10 mg/kg cabazitaxel in combination with vehicle or 25 mg/kg ritonavir (n = 6).

In Figure 23 plots are shown presenting plasma concentrations of oral cabazitaxel administered at 10 mg/kg to Cyp3aXAV mice measured in preclinical boosting studies.

In Figure 24 plots are shown presenting plasma concentration of oral ritonavir administered in preclinical boosting studies.

In Figure 25 a schematic representation of the structural PK model is shown for oral cabazitaxel co-administered with ritonavir in Cyp3aXAV mice.

In Figure 26 goodness of fit plots are shown for the ritonavir PK model in mice.

In Figure 27 plots are shown presenting prediction-corrected visual predictive check for the ritonavir model. The solid and dashed lines represent the median, 5th and 95th percentiles of the observations. The shaded area's represent the 95% confidence intervals of the 500 simulated datasets.

In Figure 28 goodness of fit plots are shown for the cabazitaxel PK model in mice.

In Figure 29 plots are shown presenting visual predictive checks for the cabazitaxel PK model stratified by boosting strategy.

In Figure 30 a schematic representation of the composite structural PK model is shown for oral cabazitaxel co-administered with ritonavir in humans.

In Figure 31 plots are shown presenting $AUC_{0\text{-inf}}$ calculated based on Monte Carlo simulations (n=1000) of IV cabazitaxel (25 mg/m$^2$) simulated for a typical patient of 1.9 m$^2$ and oral cabazitaxel at different dose levels of 1 - 40 mg concomitant with 100 mg ritonavir.

**Detailed description**

Cabazitaxel administered intravenously as a chemotherapeutic agent is approved and in use for the treatment of hormone-refractory metastatic prostate cancer. Patients with hormone-refractory metastatic prostate cancer normally start chemotherapy with docetaxel, because this compound showed an overall survival benefit in patients suffering from it (Petrylak et al. N Engl J Med. 2004). However, eventually patients can develop resistance to docetaxel via multiple pathways, including perhaps expression of the cytochrome P450 enzyme 3A4 (CYP3A4) in the tumor or upregulation of the multidrug efflux protein P-glycoprotein (P-gp/ABCB1) (Seruga et al. Nat Rev Clin Oncol. 2011; Ikezoe et al. Cancer Res. 2004). Castration-resistant prostate cancer patients with progression after docetaxel have the option to switch to cabazitaxel in combination with prednisolone (FDA, U.S.F.a.D.A., Prescribing information Jevtana (cabazitaxel). 2010; EMA, Assessment Report for Jevtana (cabazitaxel). 2011). Cabazitaxel exerts cytotoxicity in tumor cells which had previously developed resistance against docetaxel (de Bono et al. Lancet. 2010; Vrignaud et al. Clin Cancer Res. 2013). The exact mechanism behind this additional cytotoxic effect over docetaxel is still unclear, but it is possibly due to a greater tumor cell penetration of cabazitaxel, also as the loss of hydroxyl groups further reduces polarity (de Bono et al. Lancet. 2010; Vrignaud et al. Clin Cancer Res. 2013).

Similar to docetaxel and paclitaxel, cabazitaxel is extensively metabolized in the liver and intestine by CYP3A4/5, for approximately 80-90%, and to a lesser extent by CYP2C8. During the metabolism of cabazitaxel approximately 20 metabolites are formed in vivo, from which three metabolites are pharmacodynamically active (FDA, U.S.F.a.D.A., Prescribing information Jevtana (cabazitaxel). 2010; EMA, Assessment Report for Jevtana (cabazitaxel). 2011). These active metabolites are two O-demethylated derivatives of cabazitaxel, referred to as DM1 and DM2 herein, and docetaxel itself. DM1, DM2, and docetaxel showed in vitro cytotoxic effects with a comparable IC50 to cabazitaxel itself (EMA, Assessment Report for Jevtana (cabazitaxel). 2011). However, none of the active metabolites accounted individually for more than 10% of the systemic exposure to the parent drug EMA, Assessment Report for Jevtana (cabazitaxel). 2011. The conversion of cabazitaxel to these three metabolites is primarily mediated by CYP3A4/5, as is the further degradation of docetaxel (EMA, Assessment Report for Jevtana (cabazitaxel). 2011; Sarantopoulos et al. Cancer Chemother Pharmacol. 2014). Therefore, extensive metabolism by CYP3A4/5 will reduce the bioavailability, especially oral availability, of cabazitaxel,

In a pharmaceutical composition in accordance with the invention, the pharmaceutical composition comprises an effective amount of cabazitaxel for use in a combination treatment of cancer in a patient. The combination treatment further comprises a CYP3A inhibitor. The cabazitaxel may be in the form of an oral formulation. Oral therapy would be more patient-friendly and less costly, and it might allow metronomic therapy and there are no injection site reactions. Another advantage of orally administered cabazitaxel is that there is no indication any more for the necessary premedication with dexamethasone, which patients normally receive before an intravenous cabazitaxel dose to prevent hypersensitivity reactions. The CYP3A inhibitor helps to enhance the plasma exposure of cabazitaxel, thereby improving the bioavailability of it, by inhibiting the extraction and metabolism of cabazitaxel in the gastrointestinal tract. Also, oral formulation allows the avoidance of use of standard premedication with high dose dexamethasone, which is recommended in every cycle during the standard of care treatment for cabazitaxel.

As used herein, the oral administration of cabazitaxel to a subject includes any route through the mouth of introducing or delivering to a subject the agent to perform its intended function. Suitable pharmaceutical compositions for oral administration includes liquids, tablets or capsules. Capsules and tablets may have an enteric coating, such that cabazitaxel is released from the capsules or tablets in the intestine. Capsules and tablets may be formulated in an extended release formulation such that cabazitaxel is released over an extended period, e.g. several hours or more, e.g. during the time spent in the intestinal tract. Tablets and capsules may thus be formulated such that the agent is released therefrom gradually. Tablets and capsules may be formulated such that the agent is released in the stomach or intestine. Tablets and capsules may be formulated such that the agent is released in the stomach and intestine. Administration includes self-administration and the administration by another.

Pharmaceutical compositions of this invention may comprise cabazitaxel, or pharmaceutically acceptable salts and esters thereof, and/or a CYP3A inhibitor, such as ritonavir, (or pharmaceutically acceptable salts and esters thereof) together with any pharmaceutically acceptable carrier, adjuvant or vehicle. Suitable preparations and/or pharmaceutical compositions for oral administrations include formulations as described in WO2009027644, WO2010020799 and Moes et al. Drug Deliv. Transl. Res. 2013) which are incorporated herein in its entirety by reference. Any suitable preparation for oral administration can be contemplated.

The current invention may not be restricted to oral administration of cabazitaxel. Any administration of cabazitaxel via the gastrointestinal tract may be contemplated. Hence, enteral administration can be contemplated herein instead of oral administration. Preferably, enteral administration is in the form of capsules, tablets, and suppositories. Cabazitaxel administration via a suppository may be advantageous, as bioavailability may be improved as compared with oral administration. This is because with oral administration, after passing the stomach and intestine, cabazitaxel is delivered to the liver via the portal vein. By enteral administration, the barriers that metabolize cabazitaxel in the first-pass may be avoided. Any enteral administration may suffice, as long as peak levels are avoided and effective plasma levels are obtained as described herein.

The pharmaceutical composition comprising an effective amount of cabazitaxel may also be a formulation for intravenous administration, for example intravenous infusion. The present invention improves the bioavailability of cabazitaxel in tissues such as liver and brain. Lower doses of cabazitaxel may therefore be used for intravenous administration, reducing side effects.

The combination treatment in accordance with the invention further comprises a CYP3A inhibitor. For many anticancer drugs such as cabazitaxel, cytochrome P450 represents a main oxidative drug metabolizing enzyme system. Cytochrome P450 (CYP) iso-enzymes, in particular CYP3A4, which also may include CYP3A5, (referred to as CYP3A herein) are highly expressed in the liver and intestine. Extraction and metabolism of cabazitaxel by this enzyme system in the liver and intestine play an important role in limiting bioavailability. As part of the metabolic route transporters also play a role. By the transport of compounds, such as cabazitaxel, in and out of the cell, the compound is provided as a substrate to the CYP3A4 and/or CYP3A5 enzymes. For example, the P-glycoprotein (P-gp, MDR1, ABCB1) plays a role in the metabolic route and transport of cabazitaxel. Hence, any compound that may have an effect on the metabolic route of cabazitaxel to thereby inhibit metabolizing cabazitaxel may be considered a suitable CYP3A inhibitor. Such compounds have an effect on CYP3A4 and/or CYP3A5, and on P-glycoprotein (Er-jiaWang et al, Chem. Res. Toxicol. 2001; Wacher et al., Mol Carc. 1995), or may have distinct action on either CYP3A4 and/or CYP3A5, and on P-glycoprotein (Er-jiaWang et al, Chem. Res. Toxicol. 2001). The use of a CYP3A inhibitor accordingly assisting in transporting cabazitaxel from the stomach and/or intestine to the bloodstream, by reducing and/or inhibiting CYP3A4 and/or CYP3A5 activity in the cell. The use of a CYP3A inhibitor can thus provide for increased bioavailability of cabazitaxel. Such bioavailability may be increased, while not substantially increasing the peak levels of cabazitaxel. Hence, the use of a CYP3A inhibitor allows for the use of a lower dosage of cabazitaxel as effective plasma levels of cabazitaxel can be increased as compared with not using a CYP3A inhibitor. Alternatively, the use of a CYP3A inhibitor allows for the use of less frequent dosing of cabazitaxel, as effective plasma levels with the area under the curve as defined herein can be more efficiently obtained as compared with not using a CYP3A inhibitor.

Suitable CYP3A inhibitors may thus have an effect on CYP3A4 and/or CYP3A5. Suitable CYP3A inhibitors may also have an effect on P-glycoprotein. Hence, a CYP3A inhibitor is defined herein as a compound capable of reducing CYP3A4 and CYP3A5 metabolism in the cell. Said compound preferably is a pharmaceutical compound. A suitable CYP3A inhibitor may be potent CYP3A inhibitors selected from the group consisting of boceprevir, claritromycine, erytromycine, indinavir, itraconazole, ketoconazole, posaconazole, ritonavir, saquinavir end voriconazole. Preferably a CYP3A inhibitor is used that has the least side effects. Selective inhibition of CYP3A4 and CYP3A5 is also highly preferred.

Preferably, the CYP3A inhibitor used in a combination treatment of the invention is ritonavir. The CYP3A inhibitor may be formulated in any dosage form that is suitable for the combination treatment. For example, the CYP3A inhibitor may be in an oral formulation, such as in a dosage form of a table or a capsule.

The dose of the CYP3A inhibitor for use in a combination treatment in accordance with the invention is equivalent to 40 to 120 mg ritonavir. One can easily establish the suitable dose for any suitable inhibitor, by determining the effect of ritonavir in a subject and establishing the dose of the chosen inhibitor that is required to achieve the same inhibition effect to 40 to 120mg ritonavir. The effect is defined as the "boosting" effect on cabazitaxel plasma levels (AUC) and/or peak plasma levels as obtained with the dose of ritonavir used. Preferably, the CYP3A inhibitor for use in a combination treatment in accordance with the invention is equivalent to 40 to 110 mg, more preferably from 50 to 110 mg, even more preferably from 60 to 110 mg, for example 100 mg ritonavir.

It was found that, surprisingly, CYP3A inhibitor used at a dose equivalent to 40 to 120 mg ritonavir is sufficient to reduce the side effects of both cabazitaxel and the CYP3A inhibitor to a minimum, whilst still getting a sufficient boosting impact on the bioavailability of cabazitaxel.

Side effects that may be controlled or reduced in the current treatment include neutropenia. Such neutropenia may be a febrile neutropenia. Neutropenia is an abnormally low concentration of neutrophils in the blood. Neutropenia is usually diagnosed by determining the absolute neutrophil count in the blood. As a reference, a healthy range of neutrophil count in the blood can be defined as having 1500 - 4000 cells per microliter of blood. Neutropenia may be diagnosed when the level of neutrophils is below 1500 cells per microliter of blood. Assays to determine neutrophil counts are widely available as part of e.g. a complete blood count analysis as part of routine laboratory testing. Accordingly, in the current invention, the incidence of neutropenia is significantly reduced in the patient population while concomitantly providing for an effective treatment of the cancer in patients. Hence, preferably, in the method of treatment of a cancer in a patient, the side effect neutropenia is controlled or reduced. Other side effects that may be controlled or reduced are thrombocytopenia, neuropathy, alopecia, fluid retention, neurotoxicity, and/or nail toxicity.

Further side effects that may be avoided by using oral administration of cabazitaxel in accordance with the invention include infusion-related reactions due to e.g. excipients (i.a. Tween-80, ethanol) used in intravenous formulations of cabazitaxel. Corticosteroids, such as dexamethasone, are used as a prophylaxis for such infusion-related reactions in current intravenous cabazitaxel treatments. By using orally administered cabazitaxel, which does not require corticosteroid prophylaxis, toxicity that may be associated with (long-term) treatment with corticosteroids may be avoided as well.

Side effects that may be controlled or reduced in the current treatment by using low dose CYP3A inhibitor include diarrhea.

In one embodiment, the pharmaceutical composition comprising an effective amount of cabazitaxel and the CYP3A inhibitor are formulated as a mixed formulation (e.g., a single composition comprising two or more active ingredients). Optionally, the mixed formulation further comprises one or more pharmaceutically acceptable carrier. The pharmaceutically acceptable carriers that may be included in the mixed formulation may be those commonly used in formulations of drugs, and may be, but not limited to, at least one selected from the group consisting of lactose, dextrose, sucrose, sorbitol, mannitol, starch, gum acacia, calcium phosphate, alginates, gelatin, calcium silicate, micro-crystalline cellulose, polyvinylpyrrolidone, cellulose, water, syrup, methyl cellulose, methylhydroxy benzoate, propylhydroxy benzoate, talc, magnesium stearate, and mineral oil. Besides these components, the mixed formulation or the pharmaceutical compositions may further include at least one selected from the group consisting of a diluent, an excipient, a lubricant, a wetting agent, a sweetener, a flavor enhancer, an emulsifying agent, a suspension agent, and a preservative.

Alternatively, the pharmaceutical composition comprising an effective amount of cabazitaxel and the CYP3A inhibitor are formulated in separate compositions. Each of the compositions may further comprise one or more pharmaceutical acceptable carrier, or one or more additional components as described above.

In one embodiment, in the method in accordance with the invention said CYP3A inhibitor is simultaneously administered with cabazitaxel. Simultaneously, as used herein, means administration (or provision) of the cabazitaxel or CYP3A inhibitor within e.g. approximately 10 minutes, more preferably within 5 minutes, most preferably within 2 minutes of the CYP3A inhibitor or cabazitaxel. Simultaneous administration is least demanding when it comes to self-administration, and helps to avoid missing doses and/or administration errors. It is understood that simultaneous administration can comprise separate administrations, e.g. in separate pharmaceutical preparations. For example, one pharmaceutical preparation suitable for oral administration comprising cabazitaxel and another pharmaceutical preparation, preferably also oral preparation, comprising the CYP3A inhibitor, such as ritonavir. It is understood that simultaneous administration can comprise one pharmaceutical preparation comprising both cabazitaxel and the CYP3A inhibitor, such as ritonavir.

Alternatively, cabazitaxel and the CYP3A inhibitor, can also be administered sequentially, e.g. one after another. Sequential administration can optimize the boosting effect of the CYP3A inhibitor, thereby enhancing bioavailability of cabazitaxel. It is understood that sequential administrations can be done by one mixed formulation, or two separate formulations. For example, one mixed formulation may be formulated such that cabazitaxel and the CYP3A inhibitor are released, or otherwise provided, sequentially. Preferably, cabazitaxel and the CYP3A inhibitor are administered, or otherwise provided, within a time frame of about 4 hours. When they are administered separately, the CYP3A inhibitor is preferably administered (or otherwise provided) before cabazitaxel, and, more preferably, within approximately 60 minutes, more preferable between 10 to 60 minutes, even more preferably between 10 to 30 minutes, most preferably between 10 to 20 minutes, for example 15 minutes, before cabazitaxel is administered (or otherwise provided).

The current inventors have established that when using a combination of a CYP3A inhibitor and cabazitaxel, sufficient cabazitaxel exposure levels can be obtained that can eradicate cancer cells while at the same time having acceptable toxicity. Sufficient cabazitaxel exposure levels can be obtained e.g. by having a sufficient dose of CYP3A and/or an adjusted dose of cabazitaxel. Preferably, the level of exposure of cabazitaxel achieved with the combination treatment of the present invention is comparable or at least comparable to a standard of care treatment for cabazitaxel.

The standard of care treatment for cabazitaxel as used herein is defined as an intravenous administration of a recommended dose of cabazitaxel. The recommended dose of cabazitaxel is usually between 10 mg/m2 and 30mg/m2 every 3 weeks, (milligrams of cabazitaxel per square meter of body surface area of a subject). A recommended dose for metastatic castration-resistant prostate cancer in patients previously treated with docetaxel-containing treatment regimen is usually 20 mg/m2 every 3 weeks. The recommended dose may also be 25 mg/m2 every 3 weeks. Cabazitaxel exposure levels of the tumor tissue can be defined herein as the area under the curve (AUC) as obtained when administering cabazitaxel intravenously and corresponding with an effective standard of care treatment for cabazitaxel. It is understood that this may not define the actual cabazitaxel level of the tissue, as cabazitaxel is measured in plasma.

The area under the curve ($AUC_{0\text{-inf}}$; ng.h/mL) represents the total drug exposure after the administration of cabazitaxel, during which time the cabazitaxel concentration in blood plasma can be measured at several timepoints, and the surface of the area under the curve can be calculated from the plotted values. Plasma levels of cabazitaxel can be measured based on methods known in the art (Hendrikx et al. J. Chrom. B. 2011), which may include liquid chromatography and mass spectrometry methods, such as e.g. described in the examples. Plasma is a blood component, it is understood that instead of measuring cabazitaxel in blood plasma, one can also determine levels of cabazitaxel in whole blood or in serum. Measurements of cabazitaxel, e.g. peak levels and area under the plasma concentration-time curve, in short area under the curve (AUC) herein are defined relative to (blood) plasma but can easily be recalculated to corresponding peak levels in whole blood or serum. In general, preferably, the $AUC_{0\text{-inf}}$ is within the range of 100 to 10000 ng.h/mL. Preferably, the $AUC_{0\text{-inf}}$ is more than 100 ng.h/mL, more than 300 ng.h/mL, more than 500 ng.h/mL, more preferably within the range of 800 to 8000 ng.h/mL. Preferably, the AUC is less than 10000 ng.h/mL. less than 8000 ng.h/mL, less than 7000 ng.h/mL, less than 6000 ng.h/mL, less than 5000 ng.h/mL less than 4000 ng.h/mL, at less than 3000 ng.h/mL. More preferably, the AUC may be within the range of 500 to 5000 ng.h/mL, for example within the range of 800 to 2000 ng.h/mL. In one embodiment of the present invention, the $AUC_{0\text{-inf}}$ of cabazitaxel is within the range of 800 to 1000 ng.h/mL Preferably, the combination treatment of the present invention achieves $AUC_{0\text{-inf}}$ equivalent to that of the standard care of treatment for cabazitaxel. Herein, plasma concentration-time curve, area under the curve, or $AUC_{0\text{-inf}}$, with reference to cabazitaxel are used interchangeably.

The cabazitaxel exposure levels obtained (as determined with $AUC_{0\text{-inf}}$ as described herein) in accordance with the invention can be comparable to a standard of care treatment, or may be selected to be higher as compared with the standard of care treatment. Hence, higher levels may be advantageous, and in the uses and methods in accordance with the invention, achieving at least comparable levels of cabazitaxel may be preferred.

An "effective amount" of cabazitaxel is an amount such that, when administered, produces desired therapeutic effect and/or desired cabazitaxel exposure. In some embodiments, the combination treatment according to the present invention comprises administering cabazitaxel by intravenous infusion at a dosage of 10 to 20 mg/m2 once every three weeks, and administering an CYP3A inhibitor equivalent to 40 to 120 mg ritonavir subsequentially or simultaneously with cabazitaxel. Cabazitaxel for use in the combination treatment of the present invention may be administered orally at a dosage of 2 to 30 mg, preferably 2.5 to 15 mg, more preferably 5 to 10 mg, for example about 7.4 mg, once every 3 weeks. In some embodiments, the combination treatment according to the present invention comprises administering cabazitaxel orally at a dosage of 2.5 to 15 mg once every three weeks, and administering an CYP3A inhibitor equivalent to 40 to 120 mg ritonavir subsequentially or simultaneously with cabazitaxel. Alternatively, it may be administered orally at a dosage of 0.5 to 10 mg, preferably 1 to 5 mg, for example 1 to 2.5 mg, once every week. The weekly dosage may be taken in one take, or be divided into two takes on the same day. For example, the weekly dosage is split so that a patient takes, for example, on one day a first dose in the morning and the second dose in the evening, once a week. This has the effect of decreasing peak levels of cabazitaxel in plasma which may aid reducing side effects, while allowing to obtain a sufficient area under the curve. It also may increase the time of systemic exposure of the drug. In a preferred embodiment, the methods, or uses, in accordance with the invention comprise cabazitaxel being administered bidaily weekly, meaning that on one day every week, cabazitaxel is administered twice, e.g. within an 8- 16 hours interval. As long as the dosing interval and/or dosage of cabazitaxel and the CYP3A inhibitor, such as ritonavir, is selected that allows to provide for cabazitaxel exposure levels in the tumor tissue which are comparable or at least comparable to a standard of care treatment for cabazitaxel, such dosing interval and/or dosage may be contemplated.

It is understood that in the methods and uses in accordance of the inventions, any additional use of compounds, including foods and further pharmaceuticals, that may have an impact on CYP3A activity, are preferably avoided as such foods may have an effect on the levels of cabazitaxel achieved in the plasma of subjects being treated. Hence, whichever potent CYP3A inhibitor is selected for the combined treatment with cabazitaxel, the further use of inhibitors of CYP3A by the subjects receiving treatment needs to be avoided as this may result in too high peak levels of cabazitaxel and/or too high area under the curves. Examples of further inhibitors that are preferably avoided are e.g. HIV Antivirals: indinavir, nelfinavir and saquinavir; Anti-microbial agents: clarithromycin, itraconazole, ketoconazole, nefazodone, telithromycin, erythromycin, fluconazole, chloramphenicol, ciprofloxacin, norfloxacin and voriconazole; Cardiac agents: verapamil, diltiazem, cimetidine and miodarone; other agents such as fluvoxamine; and also foods, such as star fruit and grapefruit juice. Conversely, preferably in the methods and uses of the invention, the use of compounds, including foods and further pharmaceuticals, that may induce CYP3A activity in the subjects receiving treatment, is preferably avoided as well, as such use may result in lower peak levels of cabazitaxel in plasma. Inducers of CYP3A that are preferably avoided are: HIV Antivirals: efavirenz and nevirapine; Other agents such as: barbiturates, carbamazepine, modafinil, nevirapine, oxcarbazepine, phenobarbital, phenytoin, pioglitazone, rifabutin, rifampicin and also St. John's wort.

While CYP3A activity, in e.g. the liver and in the intestine, can have an effect on the exposure levels that are obtained in the blood after oral administration of cabazitaxel, which can be controlled by the use of CYP3A inhibitors and/or selecting a suitable cabazitaxel dosage, there may also be other unknown causes that have an effect on the exposure levels of cabazitaxel that can be obtained after administration of cabazitaxel. Increasing the cabazitaxel dose in patients having increased clearance of cabazitaxel to obtain higher exposure levels of cabazitaxel could be beneficial to such patients. However, increasing the cabazitaxel dose through the use of intravenous administrations of cabazitaxel will provide for an unacceptable high peak concentration in blood plasma, making this unacceptable with a standard of care treatment. In contrast, by using the pharmaceutical composition for combination treatment in accordance with the invention, exposure levels of cabazitaxel can be well controlled avoiding unacceptable high peak concentrations. Hence, the inventors have established that the dose of cabazitaxel and/or the dose of the CYP3A inhibitor used can be selected such that the exposure levels obtained with cabazitaxel can be rendered highly efficacious for the treatment of a cancer and provide for an acceptable toxicity to subjects. Thus, the present invention provides cabazitaxel for use in a combination treatment for cancer, whereby the dose of the CYP3A inhibitor equivalent to 40 to 120 mg ritonavir is sufficient to obtain cabazitaxel exposure levels of the tumor tissue which are comparable to a standard of care treatment for cabazitaxel.

Preferably, provided is cabazitaxel or the CYP3A inhibitor for use in accordance with the invention as described herein, wherein the cancer is a solid tumor. Preferably, the solid tumor is a non-small-cell lung cancer, a gastric cancer, a breast cancer, a head and neck cancer or a prostate cancer. Said solid tumors being preferred because cabazitaxel has been shown to be highly efficacious in these cancers, with the combination treatment of cabazitaxel and a CYP3A inhibitor described herein providing for improved and/or acceptable toxicity to patients having these cancers.

Most preferably, said cancer is a prostate cancer. The treatment of prostate cancer can involve the use of hormonal therapy, e.g. using androgen deprivation therapy. The prostate cancer may not respond to hormonal therapy, such prostate cancer termed hormonal refractory prostate cancer (HRPC). The prostate cancer may respond to hormonal therapy, such a prostate cancer termed hormonal sensitive prostate cancer (HSPC). Such patients may also have metastases, or may develop metastases during treatment. Such cancers referred to as mHRPC or mHSPC (m indicating metastatic). A prostate cancer treatment may include castration. In any case, prostate cancer generally involves the reduction of testosterone in the body to very low levels. In prostate cancer treatments that involves the use of hormonal therapy, which involves androgen inhibitors, reduced cabazitaxel plasma levels by increased clearance of cabazitaxel may be anticipated, but such an increased clearance may need first to be established after hormonal therapy has commenced. The dosage of cabazitaxel and/or CYP3A inhibitor may therefore be adjusted in the early phase of the treatment of prostate cancer to compensate for relatively lower plasma levels in such patients as compared with patients with mCRPC. Conversely, plasma levels of cabazitaxel may need first to be established, e.g. by administering a first dose of cabazitaxel combined with a CYP3A inhibitor in accordance with the invention and determining plasma concentrations (such as AUC) of cabazitaxel, to confirm that the same doses as suitable for mCRPC can be administered. In any case, the dosages suitable for mCRPC may also be suitable for HSPC, HRPC, mHRPC, mHSPC or CRPC (i.e. non-metastatic). In a preferred embodiment, dosages suitable for mCRPC are also selected for the treatment of mHSPC.

In a preferred embodiment, the prostate cancer is a metastatic castration-resistant prostate cancer (mCRPC). Hence, in a further embodiment, in accordance with the invention, a method is provided for the treatment of a metastatic castration-resistant prostate cancer (mCRPC), comprising administering an effective dose of cabazitaxel, in combination with a CYP3A inhibitor, whereby the dose of the CYP3A inhibitor is equivalent 40 to 120 mg ritonavir.

The pharmaceutical composition and the CYP3A inhibitor for the combination treatment in accordance with the invention may be used as a first line therapy and/or treatment of a patient previously responded to treatment with an anti-cancer therapy, such as docetaxel and/or paclitaxel. Preferably, the combination treatment of the invention is used when the patient has developed resistance to first line anti-cancer therapy. Resistance to first line therapy is not only common but also expected. General mechanisms of drug resistance include, for example, P-glycoprotein upregulation, host-tumor-drug interactions, or gene mutations. Cabazitaxel provides an effective second line therapy due to its reduced transport by ABCB1 P-glycoprotein.

The current invention also provides for means and methods to determine suitable dosages of cabazitaxel and/or the CYP3A inhibitor, and/or monitoring that suitable dosages are used throughout the treatment.

Accordingly, the current invention also provides for a method for the treatment of a cancer in a patient, comprising:

- determining the activity of CYP3A in the patient
- optionally, comparing the activity of CYP3A to a reference level;
- determining a CYP3A inhibitor daily dosage within the range equivalent to 40 to 120 mg ritonavir, based on the activity level of CYP3A determined in the patient; and
- administering a combination of the CYP3A inhibitor at the determined dosage and a pharmaceutical composition comprising an effective amount of cabazitaxel,

As the activity of CYP3A in a patient may have an effect on the dosage of cabazitaxel that is to be administered to obtain sufficient exposure levels, determining the activity of CYP3A may have the advantage that possible variation between subjects can be taken into account. This can be done before the treatment commences. The CYP3A activity in a subject can be determined by any known means, but also by measuring plasma levels of ritonavir or other indirect methods. By knowing before treatment commences what an appropriate dose of the CYP3A inhibitor is, from the initiation of treatment, immediately the desired exposure levels of cabazitaxel in the subject can be obtained. Hence, in a preferred embodiment, in a method in accordance with the invention the steps of determining the activity, subsequent optional comparison step, and determining dosage step for the CYP3A inhibitor, are carried out prior to the first administration of the combination of the CYP3A inhibitor and cabazitaxel. More preferably, cabazitaxel is administered at a pre-determined dosage. When for example the CYP3A activity is relatively high before treatment, the dosage of the CYP3A inhibitor can be selected to be relatively higher, and when the CYP3A activity is low, the dosage can be selected to be relatively lower. It is understood that the CYP3A activity may also change during the treatment with the combination of oral cabazitaxel and the CYP3A inhibitor. For example, when the cancer that is the subject of the therapy comprises substantial CYP3A activity (Hendrikx et al. Int J Cancer. 2015; Ikezoe et al. Cancer Res. 2004), in treatment, because of the reduction of the cancer, the CYP3A activity in a subject may reduce as well, requiring less of a CYP3A inhibitor to maintain an efficacious level of cabazitaxel. Accordingly, in this method of treatment, the steps of the method can be carried out during the treatment comprising the administration of the combination of the CYP3A inhibitor and cabazitaxel. Hence, in a further embodiment, in the method of treatment involving determining the CYP3A activity in a subject, when the activity of CYP3A is increased during treatment, the dosage of the CYP3A inhibitor is increased, and wherein when the activity of CYP3A is decreased during treatment, the dosage of the CYP3A inhibitor is maintained or reduced as compared with the previous dosage administered. This way, optimal exposure levels in subjects can be obtained avoiding too high or too low levels of cabazitaxel after oral administration of cabazitaxel and a CYP3A inhibitor.

It is understood that as described above, the activity of CYP3A and cabazitaxel plasma levels may be carried out in treatment only, i.e. after the first administration of the combination of CYP3A inhibitor and cabazitaxel. It is also understood that the method of treatment considers first CYP3A activity of a subject, resulting in selecting first dosages of cabazitaxel and CYP3A inhibitor, followed by subsequent in treatment monitoring of CYP3A activity and/or cabazitaxel plasma levels to determine suitable dosages for CYP3A inhibitor and/or orally formulated cabazitaxel.

In further embodiments, kits are provided that are for use in the methods and uses as described herein for the combination treatment of cabazitaxel and the CYP3A inhibitor. The kit comprises a pharmaceutical composition comprising an effective amount of cabazitaxel and a CYP3A inhibitor. In one embodiment, the cabazitaxel is formulated as an oral formulation, and/or the CYP3A inhibitor is ritonavir. Preferably, the kit is for the treatment of a solid tumor, in particular non-small cell-lung cancer, gastric cancer, a breast cancer, a head and neck cancer or a prostate cancer, more in particular mCRPC. In still another embodiment, a kit is provided comprising a pharmaceutical composition comprising an effective amount of cabazitaxel and a CYP3A inhibitor, wherein said kit is for use in a combination treatment as defined in any one of the methods and uses in accordance with the invention as described herein.

Pharmaceutical compositions of this invention may comprise cabazitaxel, or pharmaceutically acceptable salts and esters thereof, and/or a CYP3A inhibitor, such as ritonavir, (or pharmaceutically acceptable salts and esters thereof) together with any pharmaceutically acceptable carrier, adjuvant or vehicle.

Pharmaceutical compositions for use in a combination treatment of the invention, may be used with one or more anti-cancer agent, and/or one or more radiosensitizing agent, and/or one or more immunotherapy agent. An anti-cancer agent is any substance that kills cancer cells, for example a cytotoxic agent, such as a chemotherapy cytotoxic agent. For example, the one or more anti-cancer agent may be selected from the group consisting of alkylating drugs, cytotoxic antibiotics, antimetabolite drugs, vinca alkaloids, photodynamic drugs and therapies, platinum drugs, taxanes, topoisomerase inhibitors, tyrosine kinase inhibitors, targeted agents such as monoclonal antibodies, and other targeted anti-cancer drugs. Optionally, the pharmaceutical composition in accordance with the invention may be used in combination with radiotherapy and/or chemotherapy after surgery.

As used in the description of the invention, clauses and clauses appended claims, the singular forms "a", "an" and "the" are used interchangeably and intended to include the plural forms as well and fall within each meaning, unless the context clearly indicates otherwise. Also, as used herein, "and/or" refers to and encompasses any and all possible combinations of one or more of the listed items, as well as the lack of combinations when interpreted in the alternative ("or").

As used herein, the term "about" will be understood by persons of ordinary skill in the art and will vary to some extent depending upon the context in which it is used. If there are uses of the term which are not clear to persons of ordinary skill in the art given the context in which it is used, "about" will mean up to plus or minus 10% of the particular term.

**Examples**

Materials

*Cabazitaxel*

A clinical cabazitaxel formulation (Jevtana) was used and was obtained from the hospital pharmacy of the Antoni van Leeuwenhoek hospital in Amsterdam, The Netherlands.

*Ritonavir*

Ritonavir was supplied by Sequoia Research Products (Pangbourne, United Kingdom).

*Sodium chloride*

Sodium chloride (0.9%, w/v) was purchased from B. Braun Medical Supplies (Melsungen, Germany).

*Isoflurane*

Isoflurane was supplied by Virbac Nederland (Barneveld, The Netherlands), and heparin (5000 IU·mL-1) was obtained from Leo Pharma (Breda, The Netherlands).

*Bovine Serum Albumin*

Bovine Serum Albumin (BSA) Fraction V was purchased from Roche Diagnostics GmbH (Mannheim, Germany).

*Other reagents and chemicals*

All other reagents and chemicals were obtained from Sigma-Aldrich (Steinheim, Germany).

Animals

Mice were housed and handled according to institutional guidelines in compliance with Dutch and EU legislation. All experimental animal protocols were approved by the institutional board for care and use of laboratory animals. Male wild-type, Cyp3a-/-, and Cyp3aXAV (overexpression of human CYP3A in liver and intestine of Cyp3a-/-) mice were used. All mice were of a >99% FVB genetic background, and they were used between 8 and 16 weeks of age. All mice used in the Examples, including the wild-type strain, displayed the so-called "high" Ceslc phenotype as described by Tang et al., thus abrogating a possible source of differences in plasma pharmacokinetics of cabazitaxel (van Waterschoot et al. Cancer Res. 2009). Animals were kept in a specific pathogen-free, temperature-controlled environment with a 12-hour light and 12-hour dark cycle. They received a standard diet (Transbreed, SDS Diets, Technilab-BMI, Someren, The Netherlands) and acidified water *ad libitum.* Animal welfare assessments were performed before, during, and after the experiments.

Oral drug solutions

Cabazitaxel was orally administered at a dose of 10 mg/kg of bodyweight. Commercially available cabazitaxel (Jevtana), a solution for infusion, was used to obtain the oral dosing solution. The Jevtana (10 mg/mL) solution contains 0.26 mg/mL polysorbate 80 and 15% (w/v) ethanol, and is adjusted to pH 2-6 with citric acid. This solution was further diluted 10-fold with saline (NaCl 0.9% w/v) to yield a cabazitaxel concentration of 1 mg/mL. For the main boosting experiment, ritonavir was orally administered at a dose of 25 mg/kg of bodyweight. Therefore, a stock solution of 12.5 mg/mL ritonavir was prepared in polysorbate 80/ethanol (1:1, v/v) and stored at -30°C. The stock solution was further diluted 5-fold with water to reach a 2.5 mg/mL dosing solution for oral administration. The vehicle solution only consists of polysorbate 80, ethanol and water (1:1:8, v/v/v). For the reduced ritonavir experiment, ritonavir was administered at a dose of 1 mg/kg (dosing solution 0.1 mg/mL) or 10 mg/kg (dosing solution 1 mg/mL). These dosing solutions were obtained by preparing a ritonavir stock solution of 10 mg/mL in polysorbate 80/ethanol (1:1, v/v) and dilute this further by 100- or 10-fold with water, respectively. All dosing solutions were prepared freshly on the day of the experiment.

Plasma pharmacokinetics and tissue distribution of cabazitaxel and metabolites in combination with ritonavir

Before the start of the experiments, all animals were fasted 3 to 4 hours prior to vehicle or ritonavir, and cabazitaxel oral administration to minimize absorption variation. Cabazitaxel was orally administered 15 minutes after vehicle or ritonavir administration. During the main experiment, mice received vehicle solution or ritonavir (25 mg/kg of body weight; 2.5 mg/mL dosing solution) by oral gavage using a blunt-ended needle, to male wild-type, Cyp3a-/-, and Cyp3aXAV mice. Subsequently, cabazitaxel was administered directly into the stomach by oral gavage. In the reduced ritonavir experiment, vehicle, ritonavir (1 mg/kg of body weight; 0.1 mg/mL dosing solution), or ritonavir (10 mg/kg of body weight; 1 mg/mL dosing solution) was administered to male wild-type and Cyp3aXAV mice, 15 minutes prior to the cabazitaxel administration. Blood samples were obtained from the tail vein (~50 $\mu$L per sample) at respectively 0.25, 0.5, 1, 2, 4, and 8 hours after cabazitaxel administration, using heparin-coated microvettes. At the termination time point of 24 hours, mice were anesthetized using an isoflurane evaporator with 3% isoflurane with 0.8 L/min air and 0.3 L/min oxygen. A final blood sample was taken using cardiac puncture, using Eppendorf tubes previously prepared with heparin as an anticoagulant. Subsequently, the anesthetized mice were sacrificed by cervical dislocation and the tissues of interest were collected. Brain, liver, spleen, kidney, small intestine, and testis were collected. The small intestinal content (SIC) was separately collected by removing this from the small intestinal tissue, which was subsequently rinsed with saline to remove possibly fecal residue. The blood samples were centrifuged at 9,000 $\times$ g for 6 minutes at 4°C, and the plasma fraction was isolated and stored at -30°C until analysis. Brain, liver, spleen, kidney, small intestinal tissue, SIC, and testis were homogenized in a FastPrep-24TM 5G homogenizer (M.P. Biomedicals, Santa Ana, CA, USA) for 1 min with 1, 3, 1, 2, 3, 2, and 1 mL of 2% (w/v) BSA in Milli-Q water, respectively. Until analysis of the samples, they were all stored at -30°C.

Bioanalytical assay

Concentrations of cabazitaxel and its three metabolites were measured with liquid chromatography-tandem mass spectrometry (LC-MS/MS). In the same run also the ritonavir concentrations were measured. The concentrations of cabazitaxel, docetaxel and ritonavir were quantitatively determined, whereas DM1 and DM2 were semi-quantitatively determined in plasma samples and tissue homogenates.

Pharmacokinetic calculations and statistical analysis

Pharmacokinetic parameters were estimated by non-compartmental analysis using the software package of PK solutions 2.0.2 (SUMMIT, Research Service). The area under the curve (AUC) of the plasma-concentration time curve for all the compounds was calculated using the linear trapezoidal rule without extrapolating to infinity. The peak plasma concentration (Cmax) and the time to reach peak plasma concentration (Tmax) were obtained directly from the individual raw data. For the statistical analysis and the graphs GraphPad Prism 9 (GraphPad Software Inc., La Jolla, CA, USA) was used. The statistics for the comparison of multiple groups were calculated with the one-way analysis of variance (ANOVA), whereas Sidak's post hoc correction was performed. Log-transformation before statistical analysis was applied, when variances within the groups were not homogeneously distributed. Differences were considered statistically significant when P < 0.05. All data are presented as geometric mean $\pm$ SD.

Example 1 - Pharmacokinetics

*Boosting the oral availability of cabazitaxel by coadministration of ritonavir*

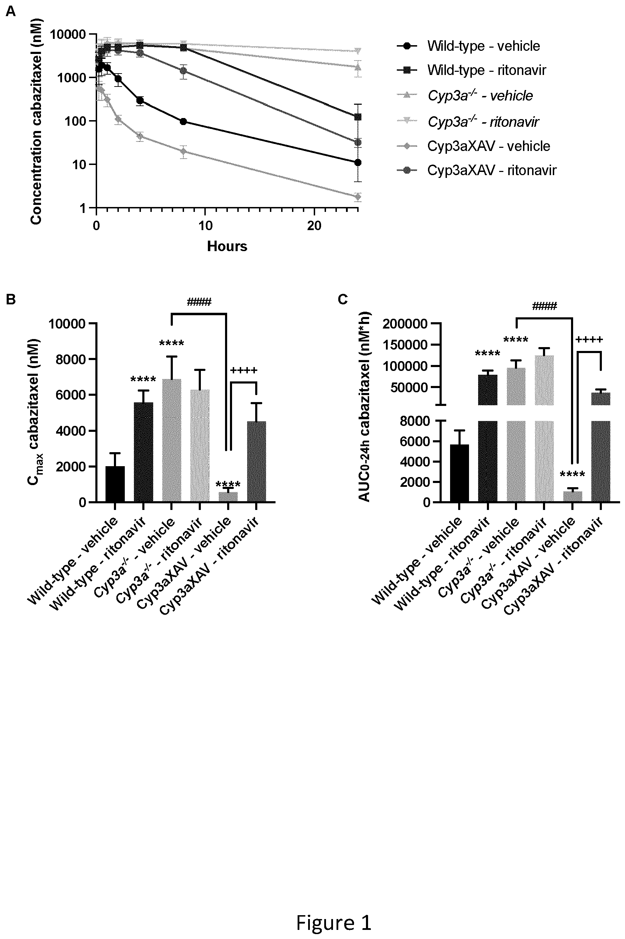

The potential influence of oral coadministration of the CYP3A inhibitor ritonavir at a dose of 25 mg/kg was evaluated in a 24-h experiment, using male wild-type, Cyp3a-/-, and Cyp3aXAV mice (transgenic mouse model; Cyp3a-/- mice with overexpression of human CYP3A4 in liver and intestine). Cabazitaxel was orally administered at a dose of 10 mg/kg of body weight, 15 minutes after oral administration of ritonavir or vehicle solution. In the vehicle groups, the plasma exposure of cabazitaxel was markedly increased in Cyp3a-deficient mice (AUC0-24h 16.8-fold and Cmax 3.4-fold, respectively) compared to wild-type (Fig. 1, Table 1). Furthermore, a profoundly reduced AUC0-24h (88.8-fold) and Cmax (12.1-fold) were observed in the transgenic Cyp3aXAV compared to Cyp3a-/- mice (Fig. 1, Table 1). Upon pretreatment with ritonavir, the time to reach the peak concentration of cabazitaxel (Tmax) was clearly prolonged for all three mouse strains, albeit least for the Cyp3a-/- mice. The plasma exposure of cabazitaxel was strongly increased by ritonavir treatment in wild-type (AUC0-24h by 13.9-fold, P < 0.0001) and Cyp3aXAV (AUC0-24h by 34.3-fold, P < 0.0001) mice compared to the vehicle groups (Fig. 1, Table 1). The Cmax was 2.8-fold enhanced in wild-type mice after coadministration of ritonavir, and 8.0-fold in Cyp3aXAV mice. Unexpectedly, the plasma exposure (AUC0-24h) of cabazitaxel in Cyp3a-/- was also slightly increased in the ritonavir compared to the vehicle group, although this was not statistically significant except for the 24-h time point (Fig. 1, Table 1). This delayed elimination in the ritonavir-treated Cyp3a-/- mice suggests that an alternative cabazitaxel clearance system may have been inhibited by ritonavir as well.

Collectively, the data indicate that mouse and human CYP3A play a pronounced role in the metabolism of cabazitaxel, and present a crucial limiting factor in its plasma exposure. Moreover, the oral availability of cabazitaxel could be dramatically enhanced by coadministration of ritonavir. Assessing the plasma curves, ritonavir treatment brought cabazitaxel plasma concentrations in wild-type mice up to the levels seen in Cyp3a-/- mice for up to 8 hours, suggesting virtually complete Cyp3a inhibition over this period. After this time point, the levels dropped off relative to those in Cyp3a-/- mice, presumably because of reduced ritonavir activity (Fig. 1A).

Table 1: Pharmacokinetic parameters of cabazitaxel in male wild-type, Cyp3a-/-, and Cyp3aXAV mice over 24 hours after oral administration of 10 mg/kg cabazitaxel in combination with vehicle or 25 mg/kg ritonavir (n = 6). Data are presented as mean $\pm$ SD. AUC0-24h, area under the plasma concentration-time curve from 0 to 24 hours; Cmax, maximum concentration in plasma; Tmax, time to reach maximum plasma concentration (h); Pbrain, brain accumulation per h; SIC, small intestinal content; SIC (% of dose), cabazitaxel in SIC as percentage of dose. ****, P < 0.0001 compared to wild-type pretreated with vehicle. ##, P < 0.01; ####, P < 0.0001 compared to Cyp3a-/- pretreated with vehicle. ++++, P < 0.0001 compared to Cyp3aXAV pretreated with vehicle.

| Parameter | Type of pretreatment and genotype | | | | | |
|---|---|---|---|---|---|---|
| | Vehicle | | | Ritonavir | | |
| | Wild-type | *Cyp3a*$^{-/-}$ | Cyp3aXAV | Wild-type | *Cyp3a*$^{-/-}$ | Cyp3aXAV |
| $AUC_{0-24h}$, nM.h | 5683 $\pm$ 1369 | 95650 $\pm$ 17224**** | 1078 $\pm$ 313****/#### | 78864 $\pm$ 10323**** | 126013 $\pm$ 16452 | 36920 $\pm$ 7975++++ |
| Fold change $AUC_{0-24h}$ | 1.00 | 16.83 | 0.19 | 13.88 | 22.17 | 6.50 |
| $C_{max}$, nM | 2019 $\pm$ 728 | 6884 $\pm$ 1282**** | 568 $\pm$ 247****/#### | 5586 $\pm$ 667**** | 6305 $\pm$ 1097 | 4539 $\pm$ 1010++++ |
| Fold change $C_{max}$ | 1.00 | 3.41 | 0.28 | 2.77 | 3.12 | 2.25 |
| $T_{max}$, h | 0.71 $\pm$ 0.33 | 2.08 $\pm$ 1.56 | 0.33 $\pm$ 0.13 | 3.83 $\pm$ 2.40 | 3.67 $\pm$ 2.58 | 1.50 $\pm$ 0.55 |
| $C_{brain}$, pmol/g | 19.00 $\pm$ 9.92 | 575.5 $\pm$ 439.7**** | 3.44 $\pm$ 1.91****/#### | 403.2 $\pm$ 111.6**** | 841.2 $\pm$ 170.6 | 206.4 $\pm$ 53.56++++ |

(continued)

| Parameter | Type of pretreatment and genotype | | | | | |
| --- | --- | --- | --- | --- | --- | --- |
| | Vehicle | | | Ritonavir | | |
| | Wild-type | *Cyp3a*$^{-/-}$ | Cyp3aXAV | Wild-type | *Cyp3a*$^{-/-}$ | Cyp3aXAV |
| Fold change $C_{brain}$ | 1.00 | 30.29 | 0.18 | 21.22 | 44.28 | 10.87 |
| Brain-to-plasma ratio | 1.169 ± 0.487 | 0.231 ± 0.111**** | 1.516 ± 1.150#### | 3.198 ± 1.280* | 0.145 ± 0.025 | 4.859 ± 2.173+++ |
| Fold change ratio | 1.00 | 0.20 | 1.30 | 2.74 | 0.12 | 4.16 |
| $P_{brain}$ (h$^{-1}$) | 0.0022 ± 0.0006 | 0.0041 ± 0.0029* | 0.0021 ± 0.0008# | 0.0035 ± 0.0006 | 0.0047 ± 0.0008 | 0.0039 ± 0.0004++ |
| Fold change $P_{brain}$ (h$^{-1}$) | 1.00 | 1.86 | 0.95 | 1.59 | 2.14 | 1.77 |
| $C_{Liver}$, pmol/g | 152.5 ± 34.1 | 7452 ± 1562**** | 21.84 ± 11.41****/#### | 1808 ± 1344**** | 16582 ± 1784## | 813.2 ± 253.5++++ |
| Fold change $C_{liver}$ | 1.00 | 48.86 | 0.14 | 11.85 | 108.71 | 5.33 |
| Liver-to-plasma ratio | 9.73 ± 2.34 | 3.28 ± 0.81**** | 8.85 ± 4.53#### | 11.20 ± 2.38 | 2.85 ± 0.21 | 17.55 ± 3.06+++ |
| Fold change ratio | 1.00 | 0.34 | 0.91 | 1.15 | 0.29 | 1.80 |
| SIC (% of dose) | 0.012 ± 0.012 | 0.375 ± 0.169 | 0.0015 ± 0.0009 | 0.047 ± 0.037 | 0.950 ± 0.380 | 0.013 ± 0.012 |
| Fold change | 1.00 | 31.25 | 0.13 | 3.92 | 79.17 | 1.08 |

Table 2: Pharmacokinetic parameters of cabazitaxel in male wild-type and Cyp3aXAV mice over 24 hours after oral administration of 10 mg/kg cabazitaxel in combination with vehicle, 1 mg/kg ritonavir or 10 mg/kg ritonavir (n = 6). Data are presented as mean ± SD. AUC$_{0-24h}$, area under the plasma concentration-time curve from 0 to 24 hours; Cmax, maximum concentration in plasma; Tmax, time to reach maximum plasma concentration (h). ****, P < 0.0001 compared to wild-type pretreated with vehicle. ++, P < 0.01; ++++, P < 0.0001 compared to Cyp3aXAV pretreated with vehicle.

| Parameter | Type of pretreatment and genotype | | | | | |
| --- | --- | --- | --- | --- | --- | --- |
| | Vehicle | | Ritonavir 1 mg/kg | | Ritonavir 10 mg/kg | |
| | Wild-type | Cyp3aXAV | Wild-type | Cyp3aXAV | Wild-type | Cyp3aXAV |
| AUC$_{0-24h}$, nM.h | 6691 ± 840 | 1241 ± 177**** | 19702 ± 3214**** | 1790 ± 458 | 72771 ± 2618**** | 12515 ± 5414++++ |
| Fold change AUC$_{0-24h}$ | 1.00 | 0.19 | 2.95 | 0.27 | 10.9 | 1.87 |
| $C_{max}$, nM | 3511 ± 452 | 850 ± 200**** | 5087 ± 917 | 1450 ± 445++ | 8228 ± 873**** | 3558 ± 1313++++ |
| Fold change $C_{max}$ | 1.00 | 0.24 | 1.45 | 0.41 | 2.34 | 1.01 |
| $T_{max}$, h | 1.00 ± 1.50 | 0.25 ± 0.00 | 033 + 0.13 | 0.33 ± 0.13 | 1.08 ± 0.49 | 0.67 ± 0.26 |

*Impact of ritonavir on cabazitaxel pharmacokinetics*

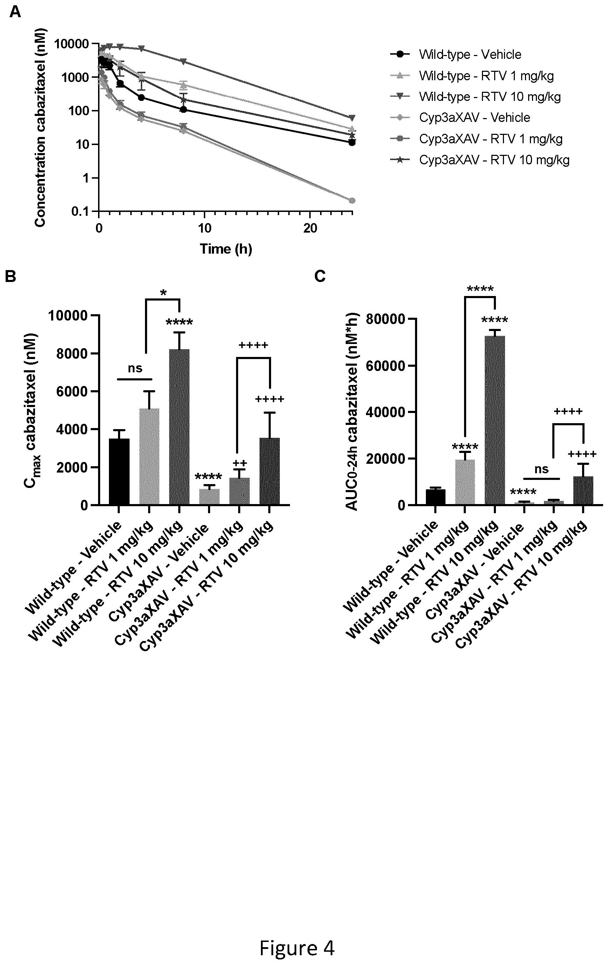

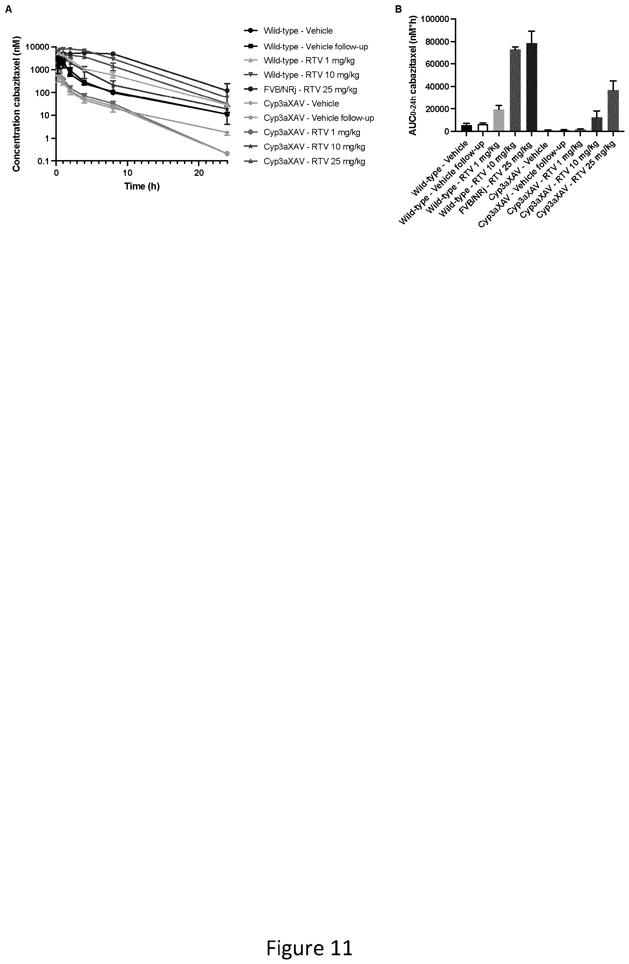

Based on the high impact of 25 mg/kg of ritonavir on the oral availability of cabazitaxel, reaching probably maximum CYP3A inhibition for several hours, the effect of lower doses of ritonavir on the plasma exposure of orally administered cabazitaxel was investigated. a follow-up experiment in wild-type and Cyp3aXAV mice was performed, in which ritonavir was administered at a dose of 1 mg/kg or 10 mg/kg before oral cabazitaxel at 10 mg/kg. In this experiment, only plasma samples were analyzed, because the effects on the tissues were mainly a reflection of the alterations in plasma of the different mouse strains. Cyp3a-/- mice were not taken along as the impact of ritonavir (25 mg/kg) in this strain was very limited (Fig. 1-3). After 24 hours, an impact of 1 mg/kg ritonavir in wild-type mice (2.9-fold increase of cabazitaxel $AUC_{0-24h}$, P < 0.0001) was still observed compared to the vehicle group (Fig. 4, Table 2). The effect of 10 mg/kg ritonavir was more noticeable, with an enhancement of the cabazitaxel AUC0-24h of 10.9-fold (P < 0.0001) in wild-type mice compared to their vehicle group and 3.7-fold (P < 0.0001) compared to wild-type mice pretreated with 1 mg/kg of ritonavir (Fig. 4, Table 2). In contrast, between the vehicle and the 1 mg/kg ritonavir group of the transgenic Cyp3aXAV mice, there was no alteration in the AUC0-24h and only a slight increase in the Cmax (1.7-fold, P < 0.05). However, with 10 mg/kg ritonavir a strong increase in the Cyp3aXAV mice was observed for the cabazitaxel AUC0-24h (10.1-fold, P < 0.0001) and Cmax (4.2-fold, P < 0.0001) compared to the mice pretreated with vehicle (Fig. 4, Table 2). Comparing the 1, 10, and 25 mg/kg ritonavir coadministrations (Fig. 11), the boosting impact on cabazitaxel plasma exposure gradually increased over the entire dose range in both wild-type and Cyp3aXAV mice (Table 2).

*Effect of ritonavir on the pharmacokinetics of the active metabolites of cabazitaxel*

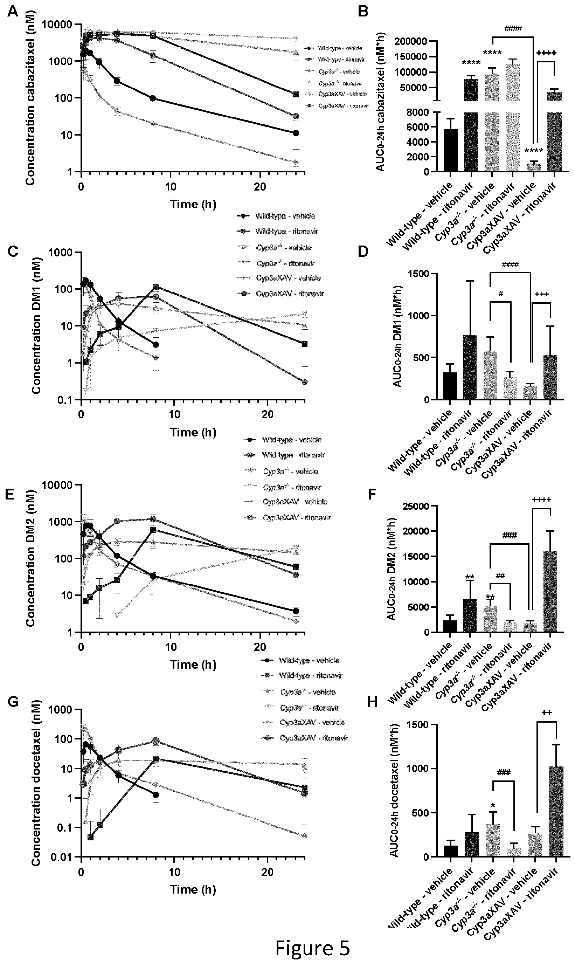

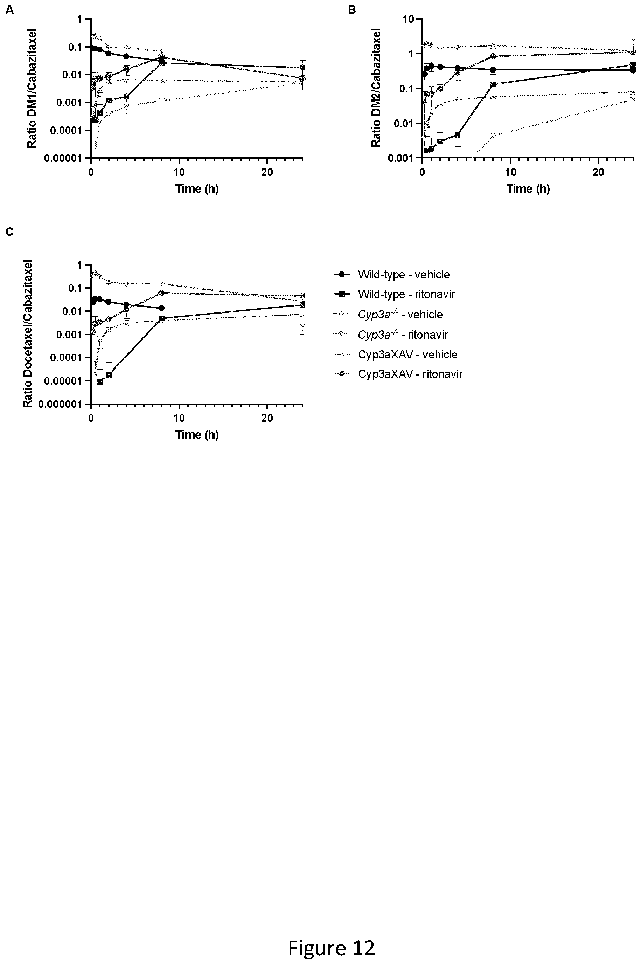

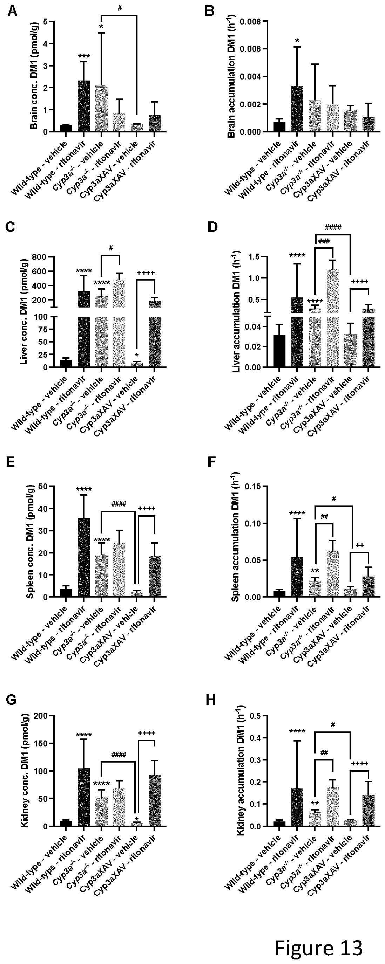

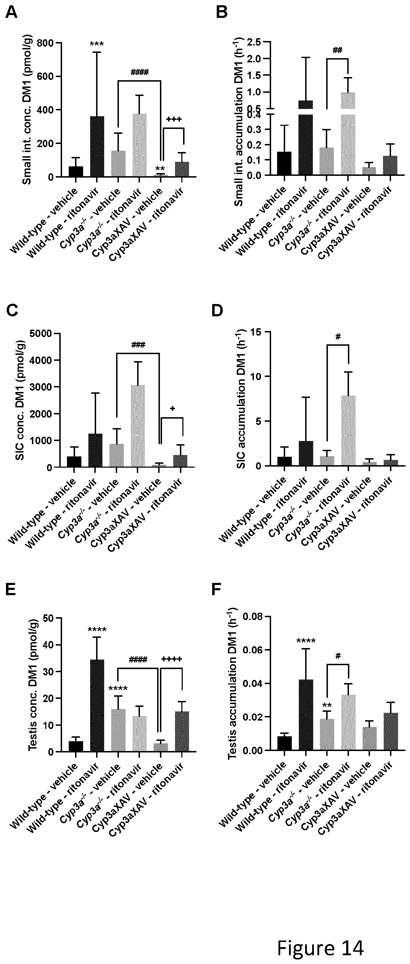

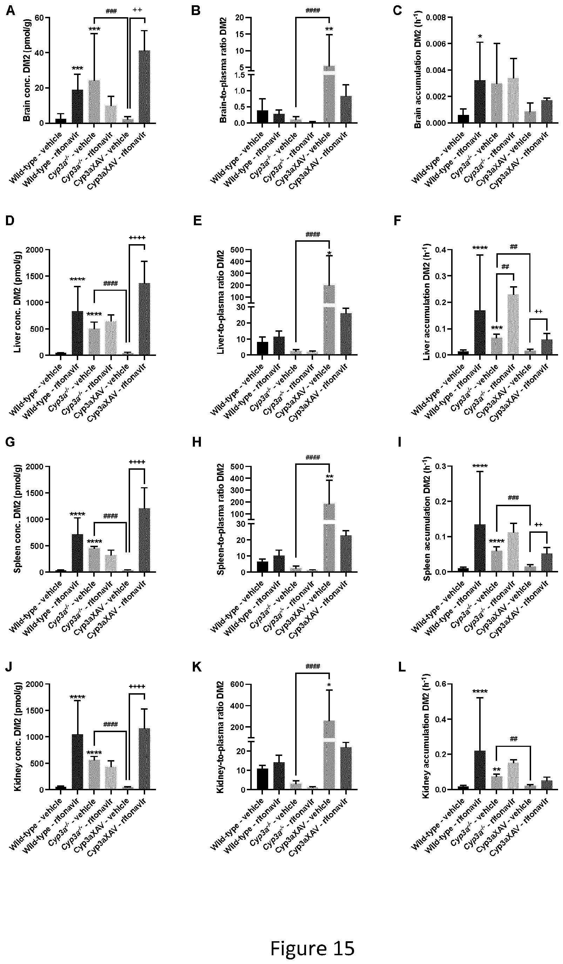

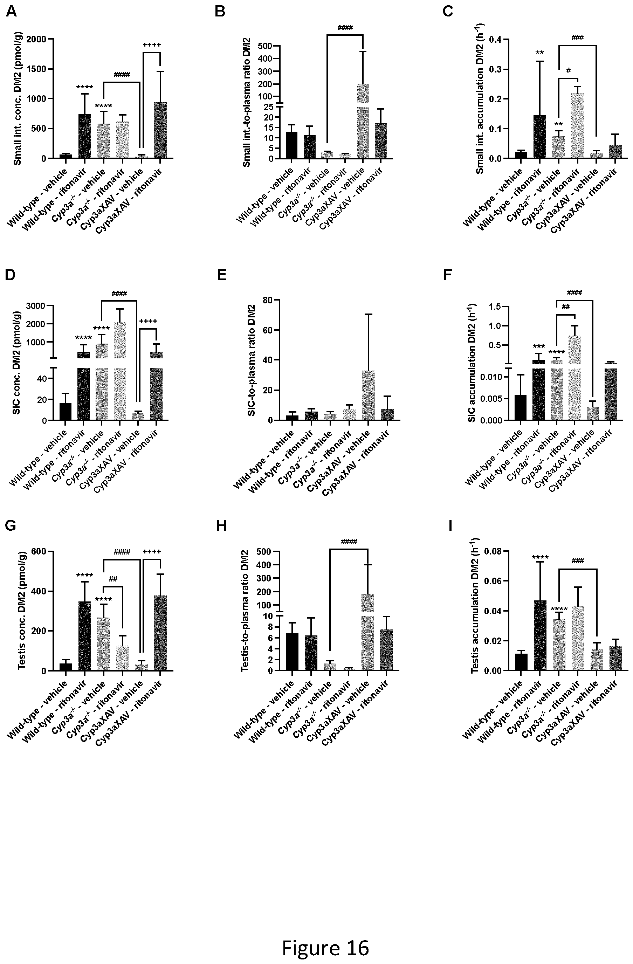

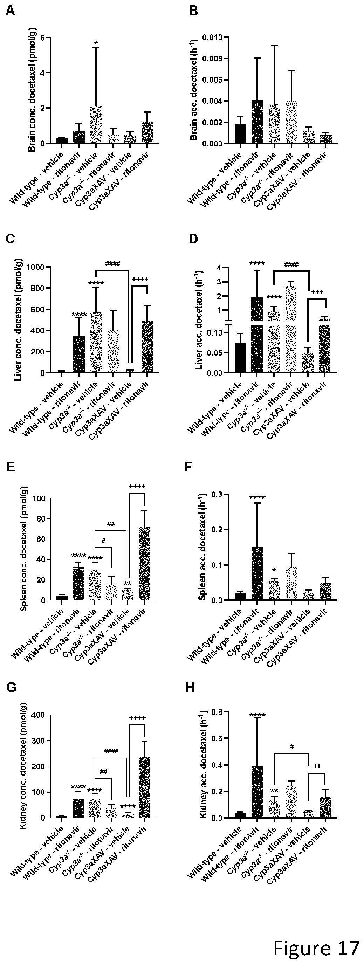

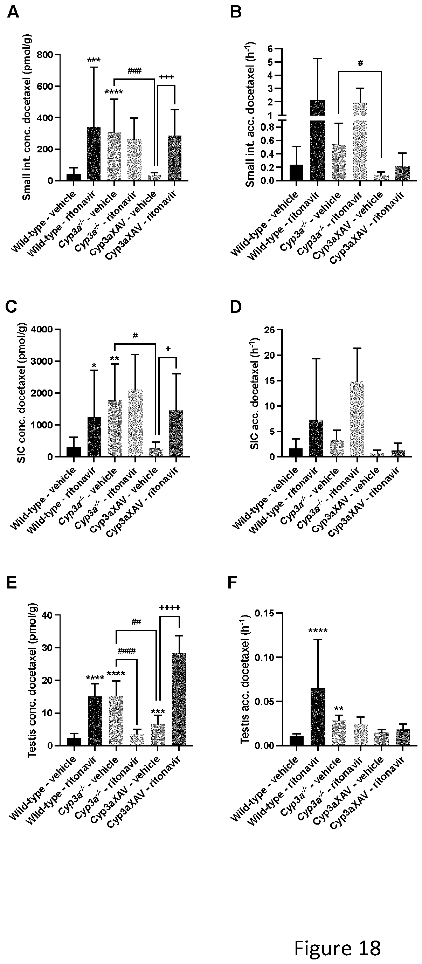

The three active metabolites of cabazitaxel, DM1, DM2 and docetaxel, were also measured in plasma and tissues (Table 3). As each compound itself can be further metabolized by CYP3A, including docetaxel, their behavior upon CYP3A inhibition or ablation might be difficult to predict. In wild-type and Cyp3a-/- mice without ritonavir pretreatment, cabazitaxel was the most abundant compound in plasma, followed by the active metabolite DM2, while DM1 and docetaxel levels were relatively low (Fig. 5). In contrast, in Cyp3aXAV mice pretreated with vehicle, DM2 reached the relatively highest plasma exposure, i.e., higher than cabazitaxel, albeit at low cabazitaxel exposure, indicating that the metabolism of cabazitaxel to DM2 was very rapid in this mouse strain. In all three mouse strains pretreated with ritonavir, the plasma concentration of cabazitaxel was higher compared to their vehicle groups, because ritonavir slowed down the biotransformation of cabazitaxel by CYP3A4. Interestingly, there was still some formation of DM1 and DM2 in Cyp3a-/- mice, suggesting that in this mouse strain there is probably another CYP enzyme(s) taking over this cabazitaxel metabolism. Furthermore, in the ritonavir groups of all three mouse strains the formation of the active metabolites took more time, with a considerably delayed Tmax. These results might indicate that CYP3A is fully inhibited at the start, but its function is slowly rehabilitating over time. This may be due to biotransformation of ritonavir itself, and/or turnover and replacement of the irreversibly inhibited Cyp3a/CYP3A4. This contrasts with the vehicle groups of the mouse strains, in which the formation of the active metabolites started directly at the beginning of the experiment, with an early Tmax. A similar conclusion could be drawn from the metabolite-to-cabazitaxel ratios, especially for DM2, which, after a quick initial rise, remained relatively constant over time in the vehicle groups, but clearly increased up to 8 hours in the ritonavir-treated groups (Fig. 12). For all the analyzed tissues (Fig. 13-18), differences in active metabolite concentrations were difficult to interpret, because these alterations were strongly driven by the changes in plasma exposure, and their plasma concentrations diverged widely at this late time point (24 hours, Fig. 5).

As DM2 was the predominant cabazitaxel metabolite, a further interpretation of the plasma results in Figure 5E is of interest, also as DM1 and docetaxel showed mostly similar behavior as DM2. The relatively higher abundance of DM2 compared to cabazitaxel in vehicle-treated Cyp3aXAV compared to wild-type mice was already mentioned, indicative of rapid conversion of cabazitaxel to DM2 by CYP3A4. Interestingly, in Cyp3aXAV mice coadministration of ritonavir dramatically increased not only the cabazitaxel exposure, but also the DM2 exposure, especially from 1-2 hours after cabazitaxel administration (Fig. 5E and F). This was likely the result of gradually recovering CYP3A4 activity, since in vehicle-treated Cyp3a-/- mice far less DM2 was formed, in spite of a much higher cabazitaxel exposure. A qualitatively similar pattern was seen for the ritonavir-treated wild-type mice (Fig. 5E), although the point in time where DM2 levels exceeded those in untreated wild-type mice was around 5-6 hours, suggesting a slower recovery of the mouse Cyp3a activity (compared to transgenic CYP3A4). Interestingly, the slow and low formation of DM2 in Cyp3a-/- mice (Fig. 5E and F) was also inhibited by ritonavir during the first 8 hours, indicating that the alternative enzyme (maybe, for example, another cytochrome) responsible for this formation is also inhibited by ritonavir. Overall, qualitatively similar profiles were observed for DM1 and docetaxel (Fig. 5C and G).

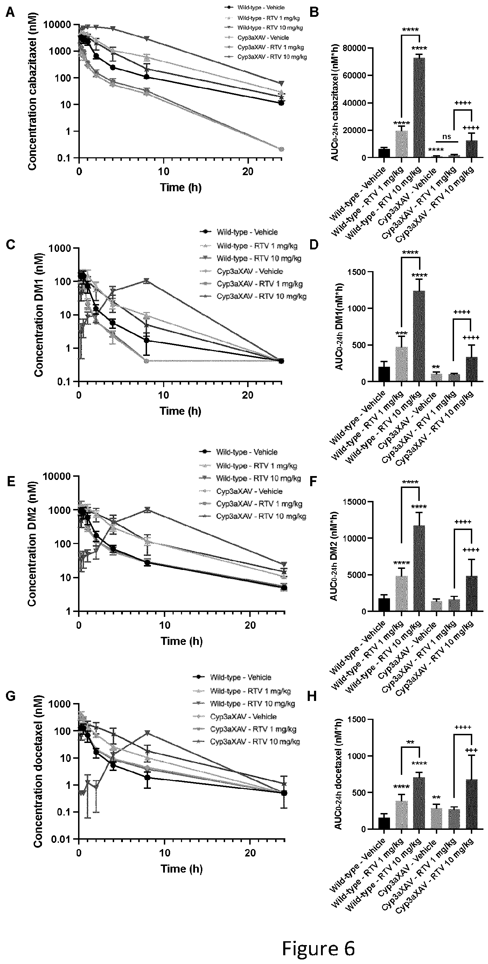

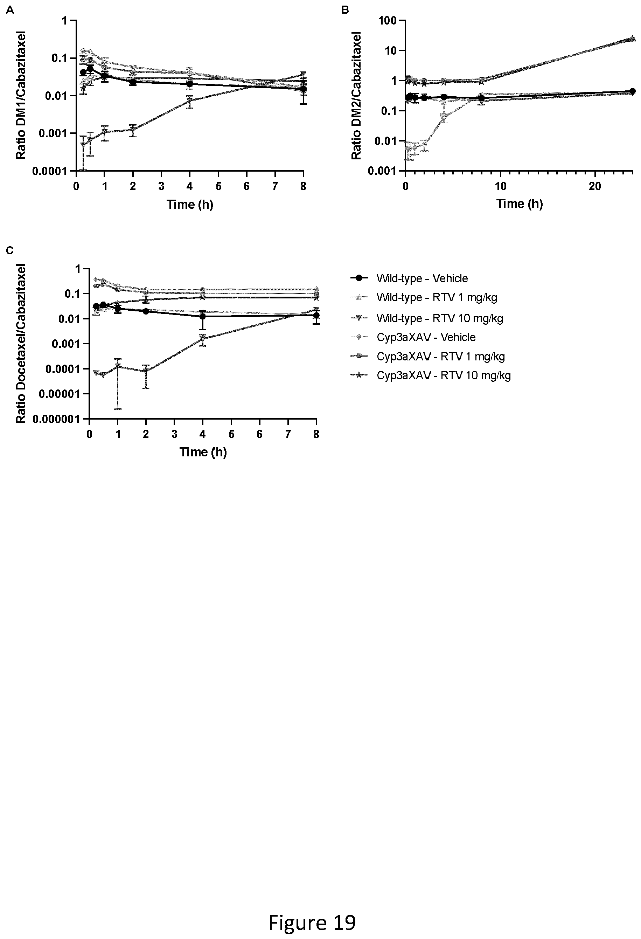

In the experiment with lower ritonavir doses, it was observed that plasma concentrations of cabazitaxel and its active metabolites in similar ranges as in the 25 mg/kg experiment (Fig. 6). As was seen for the plasma exposure of cabazitaxel itself, it seems that ritonavir at a dose of 1 mg/kg had the most impact in wild-type mice and less in the transgenic Cyp3aXAV mice (Fig. 6, Fig. 19, Table 4). No significant differences were observed in the 1 mg/kg ritonavir group of the Cyp3aXAV mice compared to their vehicle group for cabazitaxel or any of its metabolites. However, there was a significant effect of 10 mg/kg ritonavir on the formation of the active metabolites in this mouse strain (Fig. 6, Fig. 19, Table 4). It may well be that the overall abundance of transgenic CYP3A4 is considerably higher than that of the collective mouse Cyp3as, resulting in only very partial inhibition by 1 mg/kg ritonavir in Cyp3aXAV mice. Keeping this limitation in mind, and a somewhat reduced Cyp3a/CYP3A inhibition, the metabolites at 10 mg/kg ritonavir behaved qualitatively similar to those at 25 mg/kg ritonavir.

**Table 3:** Pharmacokinetic parameters of cabazitaxel and its active metabolites in male wild-type, Cyp3a-/-, and Cyp3aXAV mice over 24 hours after oral administration of 10 mg/kg cabazitaxel in combination with vehicle or 25 mg/kg ritonavir (n = 6). Data are presented as mean $\pm$ SD. $AUC_{0-24h}$, area under the plasma concentration-time curve from 0 to 24 hours; Cmax, maximum concentration in plasma; Tmax, time to reach maximum plasma concentration (h). *, P < 0.05; **, P < 0.01; ****, P < 0.0001 compared to wild-type pretreated with vehicle. #, P < 0.05; ##, P < 0.01; ###, P < 0.001; ####, P < 0.0001 compared to Cyp3a-/- pretreated with vehicle. ++, P < 0.01; +++, P < 0.001; ++++, P < 0.0001 compared to Cyp3aXAV pretreated with vehicle.

| Parameter | | Type of pretreatment and genotype | | | | | |
|---|---|---|---|---|---|---|---|
| | | Vehicle | | | Ritonavir | | |
| | | Wild-type | $Cyp3a^{-/-}$ | Cyp3aXAV | Wild-type | $Cyp3a^{-/-}$ | Cyp3aXAV |
| Cabazitaxel | $AUC_{0-24h}$, nM.h | 5683 $\pm$ 1369 | 95650 $\pm$ 17224**** | 1078 $\pm$ 313****/#### | 78864 $\pm$ 10323*** * | 126013 $\pm$ 16452 | 36920 $\pm$ 7975++++ |
| | Fold change $AUC_{0-24h}$ | 1.00 | 16.83 | 0.19 | 13.88 | 22.17 | 6.50 |
| | $C_{max}$, nM | 2019 $\pm$ 728 | 6884 $\pm$ 1282**** | 568 $\pm$ 247****/#### | 5586 $\pm$ 667**** | 6305$\pm$ 1097 | 4539$\pm$ 1010++++ |
| | Fold change $C_{max}$ | 1.00 | 3.41 | 0.28 | 2.77 | 3.12 | 2.25 |
| | $T_{max}$, h | 0.71 $\pm$ 0.33 | 2.08 $\pm$ 1.56 | 0.33 $\pm$ 0.13 | 3.83 $\pm$ 2.40 | 3.67 $\pm$ 2.58 | 1.50 $\pm$ 0.55 |
| DM1 | $AUC_{0-24h}$, nM.h nM.h | 323 $\pm$ 101 | 586 $\pm$ 161 | 156 $\pm$ 34.0#### | 770 $\pm$ 643 | 272 $\pm$ 59.7# | 529 $\pm$ 343+++ |
| | Fold change $AUC_{0-24h}$ | 1.00 | 1.81 | 0.48 | 2.38 | 0.84 | 1.64 |
| | $C_{max}$, nM | 175 $\pm$ 84.3 | 44.0 $\pm$ 10.7** | 132 $\pm$ 55.9# | 117 $\pm$ 73.0 | 21.1 $\pm$ 3.89 | 75.5 $\pm$ 20.0 |
| | Fold change $C_{max}$ | 1.00 | 0.25 | 0.75 | 0.67 | 0.12 | 0.43 |
| | $T_{max}$, h | 0.50 $\pm$ 0.27 | 4.00 $\pm$ 2.19 | 0.29 $\pm$ 0.10 | 10.67 $\pm$ 6.53 | 24.00 $\pm$ 0.00 | 6.67 $\pm$ 2.07 |
| DM2 | $AUC_{0-24h}$, nM.h | 2293 $\pm$ 1070 | 5287 $\pm$ 1211" | 1704 $\pm$ 516### | 6555 $\pm$ 3748" | 1934 $\pm$ 388## | 16073 $\pm$ 3924++++ |
| | Fold change $AUC_{0-24h}$ | 1.00 | 2.31 | 0.74 | 2.86 | 0.84 | 7.01 |
| | $C_{max}$, nM | 850 $\pm$ 494 | 303 $\pm$ 49.2* | 1056$\pm$548### | 617 $\pm$ 374 | 191 $\pm$ 40.9 | 1377 $\pm$ 312 |
| | Fold change $C_{max}$ | 1.00 | 0.36 | 1.24 | 0.73 | 0.22 | 1.62 |
| | $T_{max}$, h | 0.75 $\pm$ 0.27 | 5.00 $\pm$ 2.45 | 0.42 $\pm$ 0.13 | 10.67 $\pm$ 6.53 | 24.00 $\pm$ 0.00 | 6.67 $\pm$ 2.07 |
| Docetaxel | $AUC_{0-24h}$, nM.h | 131 $\pm$ 57.3 | 370 $\pm$ 141* | 275 $\pm$ 67.6 | 279 $\pm$ 206 | 103 $\pm$ 52.9### | 1021 $\pm$ 249++ |

(continued)

| Parameter | Type of pretreatment and genotype | | | | | |
|---|---|---|---|---|---|---|
| | Vehicle | | | Ritonavir | | |
| | Wild-type | $Cyp3a^{-/-}$ | Cyp3aXAV | Wild-type | $Cyp3a^{-/-}$ | Cyp3aXAV |
| Fold change $AUC_{0-24h}$ | 1.00 | 2.82 | 2.10 | 2.13 | 0.79 | 7.79 |
| $C_{max}$, nM | 69.6 ± 42.4 | 21.4 ± 5.63* | 228 ± 102**/#### | 22.8 ± 17.1" | 8.65 ± 4.46# | 86.2 ± 20.6 |
| Fold change $C_{max}$ | 1.00 | 0.31 | 3.28 | 0.33 | 0.12 | 1.24 |
| $T_{max}$, h | 0.67 ± 0.26 | 11.33 ± 9.93 | 0.42 ± 0.13 | 10.67 ± 6.53 | 24.00 ± 0.00 | 6.67 ± 2.07 |

**Table 4:** Pharmacokinetic parameters of cabazitaxel and its active metabolites in male wild-type and Cyp3aXAV mice over 24 hours after oral administration of 10 mg/kg cabazitaxel in combination with vehicle, 1 mg/kg ritonavir or 10 mg/kg ritonavir (n = 6). Data are presented as mean ± SD. $AUC_{0-24h}$, area under the plasma concentration-time curve from 0 to 24 hours; Cmax, maximum concentration in plasma; Tmax, time to reach maximum plasma concentration (h). *, $P < 0.05$; **, $P < 0.01$; ***, $P < 0.001$; ****, $P < 0.0001$ compared to wild-type pretreated with vehicle. +++, $P < 0.001$; ++++, $P < 0.0001$ compared to Cyp3aXAV pretreated with vehicle.

| | Parameter | Type of pretreatment and genotype | | | | | |
|---|---|---|---|---|---|---|---|
| | | Vehicle | | Ritonavir 1 mg/kg | | Ritonavir 10 mg/kg | |
| | | Wild-type | Cyp3aXAV | Wild-type | Cyp3aXAV | Wild-type | Cyp3aXAV |
| Cabazitaxel | $AUC_{0-24h}$, nM.h | 6691 ± 840 | 1241 ± 177**** | 19702 ± 3214**** | 1790 ± 458 | 72771 ± 2618**** | 12515 ± 5414++++ |
| | Fold change $AUC_{0-24h}$ | 1.00 | 0.19 | 2.95 | 0.27 | 10.9 | 1.87 |
| | $C_{max}$, nM | 3511 ± 452 | 850 ± 200**** | 5087 ± 917 | 1450 ± 445++ | 8228 ± 873**** | 3558 ± 1313++++ |
| | Fold change $C_{max}$ | 1.00 | 0.24 | 1.45 | 0.41 | 2.34 | 1.01 |
| | $T_{max}$, h | 1.00 ± 1.50 | 0.25 ± 0.00 | 0.33 ± 0.13 | 0.33 ± 0.13 | 1.08 ± 0.49 | 0.67 ± 0.26 |
| DM1 | $AUC_{0-24h}$, nM.h | 207 ± 67.8 | 109 ± 23.5** | 477 ± 138*** | 104 ± 8.33 | 1238 ± 162**** | 340 ± 161++++ |
| | Fold change $AUC_{0-24h}$ | 1.00 | 0.53 | 2.30 | 0.50 | 5.98 | 1.64 |
| | $C_{max}$, nM | 160 ± 48.4 | 135 ± 45.0 | 163 ± 61.6 | 122 ± 25.5 | 106 ± 18.0 | 101 ± 36.9 |
| | Fold change $C_{max}$ | 1.00 | 0.84 | 1.02 | 0.76 | 0.66 | 0.63 |
| | $T_{max}$, h | 0.38 ± 0.14 | 0.25 ± 0.00 | 0.54 ± 0.25 | 0.29 ± 0.10 | 7.33 ± 1.63 | 1.00 ± 0.00 |
| DM2 | $AUC_{0-24h}$, nM.h | 1789 ± 494 | 1411 ± 252 | 4855 ± 1061**** | 1627 ± 419 | 11777 ± | 4866 ± 2259++++ |
| | Fold change $AUC_{0-24h}$ | 1.00 | 0.79 | 2.71 | 0.91 | 6.58 | 2.72 |

(continued)

| Parameter | | Type of pretreatment and genotype | | | | | |
|---|---|---|---|---|---|---|---|
| | | Vehicle | | Ritonavir 1 mg/kg | | Ritonavir 10 mg/kg | |
| | | Wild-type | Cyp3aXAV | Wild-type | Cyp3aXAV | Wild-type | Cyp3aXAV |
| | $C_{max}$, nM | 983 ± 223 | 1075 ± 324 | 1189 ± 309 | 1309 ± 468 | 1019 ± 174 | 931 ± 258 |
| | Fold change $C_{max}$ | 1.00 | 1.09 | 1.21 | 1.33 | 1.04 | 0.95 |
| | $T_{max}$, h | 0.33 ± 0.13 | 0.25 ± 0.00 | 0.71 ± 0.33 | 0.29 ± 0.10 | 8.00 ± 0.00 | 1.17 ± 0.41 |
| Docetaxel | $AUC_{0-24h}$, nM.h | 158 ± 52.2 | 285 ± 54.9** | 388 ± 85.7**** | 271 ± 30.9 | 707 ± 65.8**** | 681 ± 326[+++] |
| | Fold change $AUC_{0-24h}$ | 1.00 | 1.80 | 2.46 | 1.72 | 4.47 | 4.31 |
| | $C_{max}$, nM | 119 ± 28.0 | 312 ± 96.5**** | 123 ± 41.8 | 268 ± 58.3 | 66.9 ± 7.46* | 140 ± 41.5[+++] |
| | Fold change $C_{max}$ | 1.00 | 2.62 | 1.03 | 2.25 | 0.56 | 1.18 |
| | $T_{max}$, h | 0.38 ± 0.14 | 0.25 ± 0.00 | 0.46 ± 0.10 | 0.29 ± 0.10 | 8.00 ± 0.00 | 0.92 ± 0.21 |

Example 2: Tissue disposition

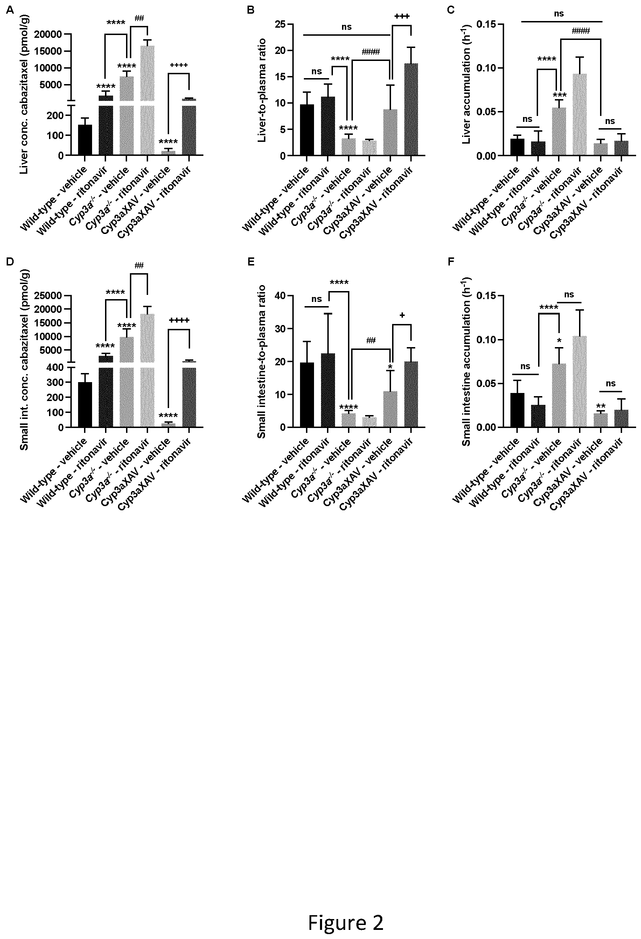

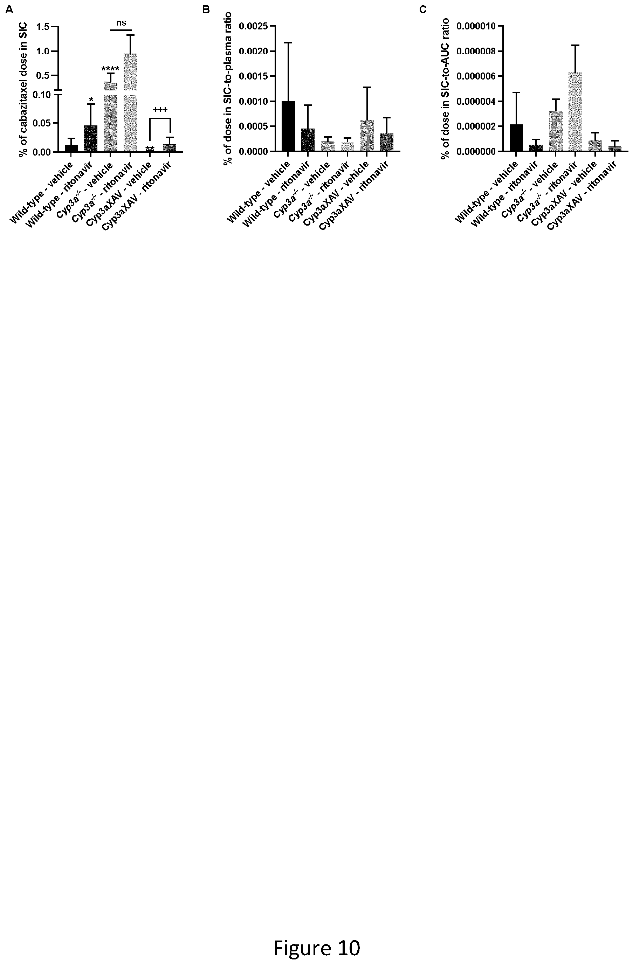

The impact of the coadministration of ritonavir on the tissue disposition of cabazitaxel at the 24-h termination time point was also assessed. Given the very large differences between the groups in overall plasma exposure and in plasma concentrations at 24 h (Fig. 1), interpretation of these data is not always straightforward. Tissue concentrations, tissue-to-plasma ratios, and tissue accumulation (tissue concentration corrected for plasma AUC0-24 h) were plotted. The last parameter is in many cases the most useful, as it takes the overall exposure into account. Disposition of cabazitaxel in liver and small intestine, the main organs for the biotransformation of cabazitaxel by CYP3A4, is important to study because of the first-pass effect and the high CYP3A4 expression in these organs. In these tissues very large differences in concentrations were observed between the different groups and strains (Fig. 2A+D). However, when considering the tissue accumulation, only relatively modest differences were observed between the strains and groups without or with ritonavir treatment (Fig. 2C+F). This suggests that the liver and small intestine concentration differences of cabazitaxel between the groups and strains were mainly driven by the differences in plasma exposure. As expected, the liver concentrations were dramatically increased (48.9-fold) in the Cyp3a-deficient compared to wild-type mice and subsequently vastly decreased in Cyp3aXAV mice (341.2-fold). The liver concentration was increased 11.9-fold (P < 0.0001) in wild-type mice pretreated with ritonavir compared to their vehicle group (Fig. 2, Table 1), but the liver-to-plasma ratio and the liver accumulation between these groups were not significantly altered. In the transgenic Cyp3aXAV mice, the liver concentration was strongly increased by ritonavir coadministration (37.1-fold, P < 0.0001, Fig. 2A, Table 1). In contrast, in the Cyp3a-/- mice ritonavir caused only a 2.2-fold enhanced liver concentration of cabazitaxel, whereas the liver-to-plasma ratio was not significantly different when compared to the vehicle group. In the small intestine tissue, a qualitatively very similar distribution pattern as for the liver was observed for all three mouse strains plus or minus ritonavir treatment for concentration, tissue-to-plasma ratio and tissue accumulation (Fig. 2C-F). The percentage of recovered cabazitaxel dose in the small intestinal content after 24 hours was quite low for all the mouse strains, and again mainly reflected the plasma concentrations of cabazitaxel (Fig. 10, Table 1).

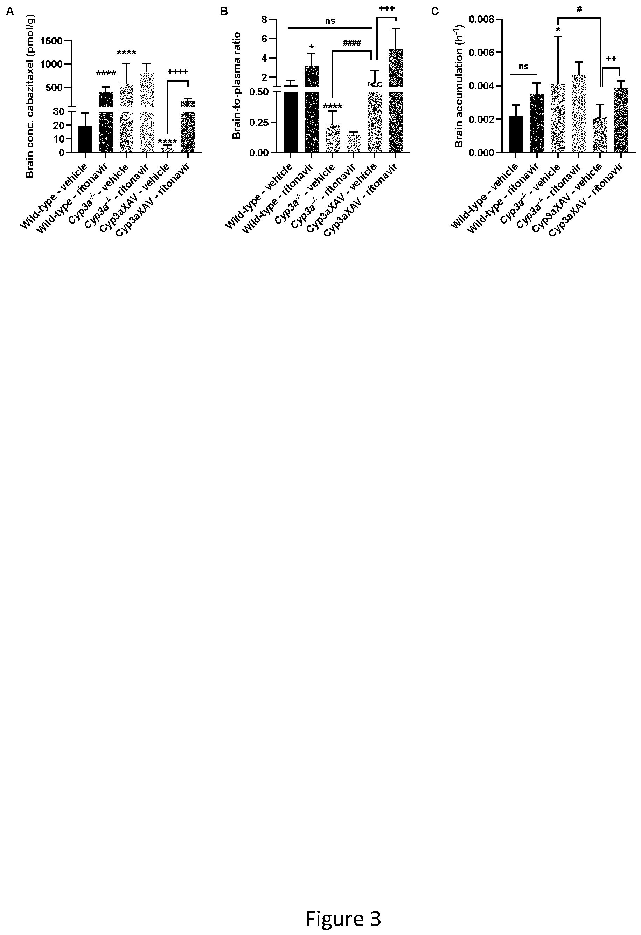

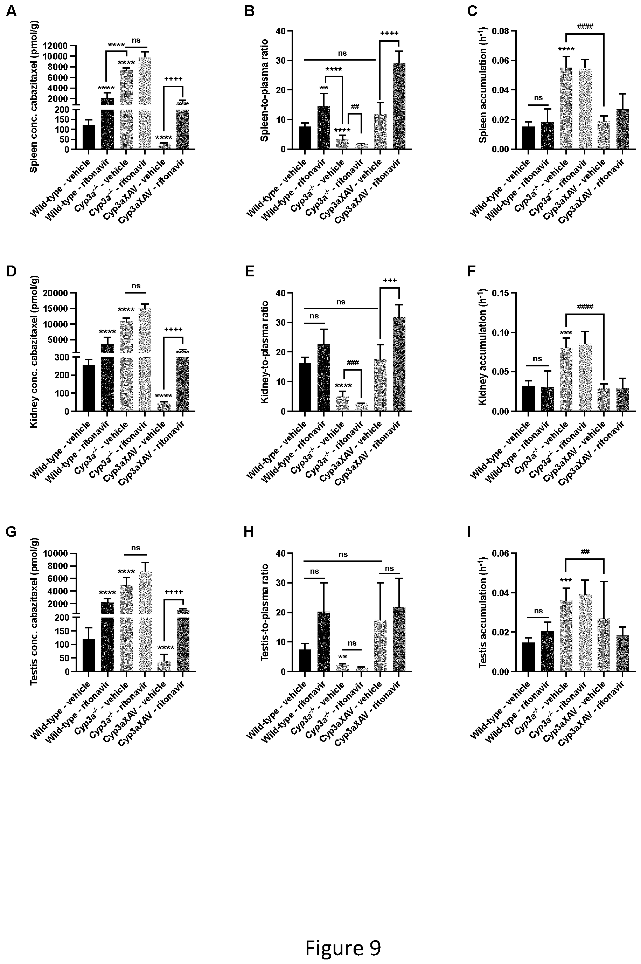

Similar considerations as for the liver and small intestine disposition apply to the brain distribution of cabazitaxel (Fig. 3A and C), given the large differences in plasma exposure and concentration between the groups. This suggests that also the brain concentration differences of cabazitaxel between the groups and strains were mainly driven by the differences in plasma exposure. The brain concentration of cabazitaxel was increased in Cyp3a-deficient mice (30.3-fold, P < 0.0001) compared to wild-type in the vehicle groups (Fig. 3A, Table 1). In the vehicle group of Cyp3aXAV mice, the brain exposure of cabazitaxel was significantly reduced (167.6-fold, P < 0.0001) compared to that in Cyp3a-/- mice. Coadministration of ritonavir led to a significant enhancement of the brain exposure to cabazitaxel in wild-type and Cyp3aXAV mice. In wild-type mice, the brain concentration was 21.2-fold (P < 0.0001) increased compared to that in wild-type mice treated with vehicle (Fig. 3A, Table 1). An even higher relative increase in brain concentration was observed in Cyp3aXAV mice pretreated with ritonavir (60.1-fold, P < 0.0001) compared to their vehicle group, in which the intrinsic brain exposure was very low (Fig. 3A, Table 1). All in all, the cabazitaxel disposition in the brain between the different groups was not much different from that in the liver and intestinal tissue (compare Fig. 2 and 3). For the other analyzed tissues (spleen, kidney, testis), while absolute cabazitaxel concentrations varied somewhat between tissues, very similar relative increase and decrease profiles were observed between the strains and treatment groups as for liver, intestine, and brain (Fig. 9). These differences are therefore likely primarily driven by the altered plasma exposure in the different strains and treatment groups, without clear differences in behavior between the different tissues.

Unexpectedly, transient early diarrhea was observed in two of the six Cyp3a-/- mice pretreated with ritonavir at a dose of 25 mg/kg, which might possibly be related to ritonavir itself. No toxicity was observed in the wild-type or Cyp3aXAV mice in both treatment groups, nor in Cyp3a deficient mice pretreated with vehicle. This transient toxicity in the ritonavir group of the Cyp3a-/- mice could be related to reduced elimination of ritonavir in these mice.

In summary, these results indicate that inhibition of CYP3A4 with ritonavir can dramatically improve tissue exposure to orally administered cabazitaxel, which could perhaps be beneficial for treatment of possible (micro-)metastases in target organs in patients. At the same time, the relative tissue distribution of cabazitaxel across a range of organs was not much altered by ritonavir treatment. Considering the differences in exposure between the mouse strains, the overall cabazitaxel exposure in Cyp3aXAV mice was much lower compared to that in wild-type mice, suggesting that the biotransformation is more rapid in the transgenic mice, possibly because of the relatively high abundance of human CYP3A4 or perhaps higher metabolic efficacy towards cabazitaxel.

Example 3: Impact of ritonavir dosages:

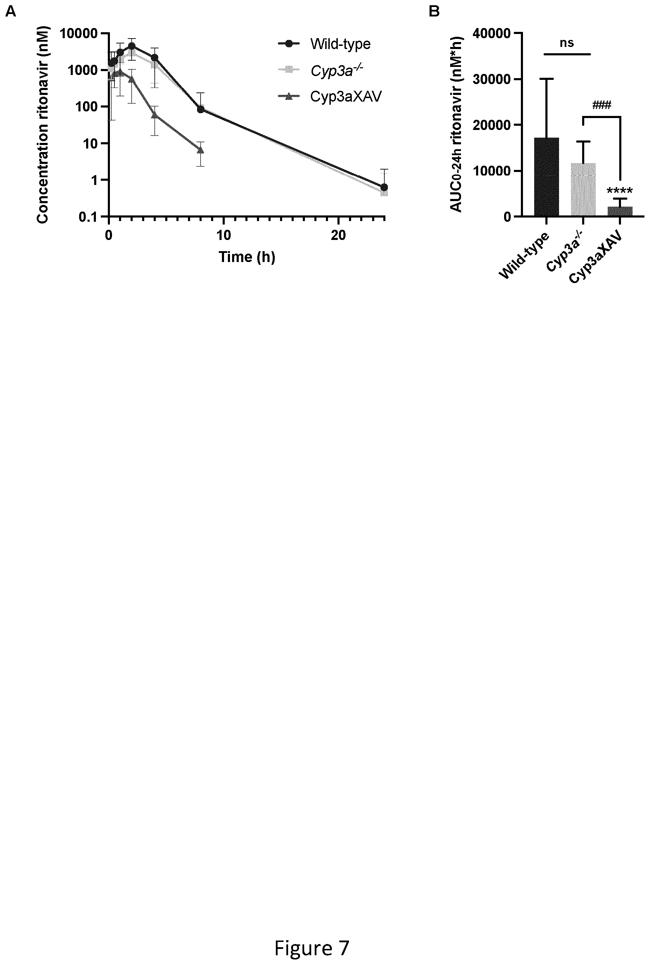

In the main and reduced-dose ritonavir experiments, the plasma concentrations of ritonavir were also analyzed. Only for the main experiment, the tissue distribution of ritonavir was also determined. Ritonavir itself is also metabolized by Cyp3a and CYP3A4. Interestingly, at 25 mg/kg ritonavir, no significant differences were observed between wild-type and Cyp3a-/- mice in plasma exposure of ritonavir, which might indicate rapid and virtually complete inhibition of the mouse Cyp3a enzymes by ritonavir at this dose, maintained for probably at least 8-10 hours. Still, the ongoing clearance of ritonavir in both strains indicates the existence of Cyp3a-independent elimination mechanism(s) for ritonavir. In contrast, in the Cyp3aXAV mice the $AUC_{0-24h}$ of ritonavir was significantly decreased by 7.9-fold (P < 0.0001) compared to wild-type, and 5.4-fold (P < 0.001) compared to Cyp3a-/- mice (Fig. 7, Table 5), starting even at early time points. Similarly, the Cmax was also reduced in the transgenic mice compared to wild-type (4.7-fold, P < 0.001), as well as Cyp3a-/- mice (3.2-fold, P < 0.01). At the last time point (24 h) ritonavir was not detectable anymore in the Cyp3aXAV mice. These data suggest that ritonavir at 25 mg/kg was not completely inhibiting the transgenic CYP3A4, even at early time points after administration, and that CYP3A4 can substantially contribute to ritonavir clearance.

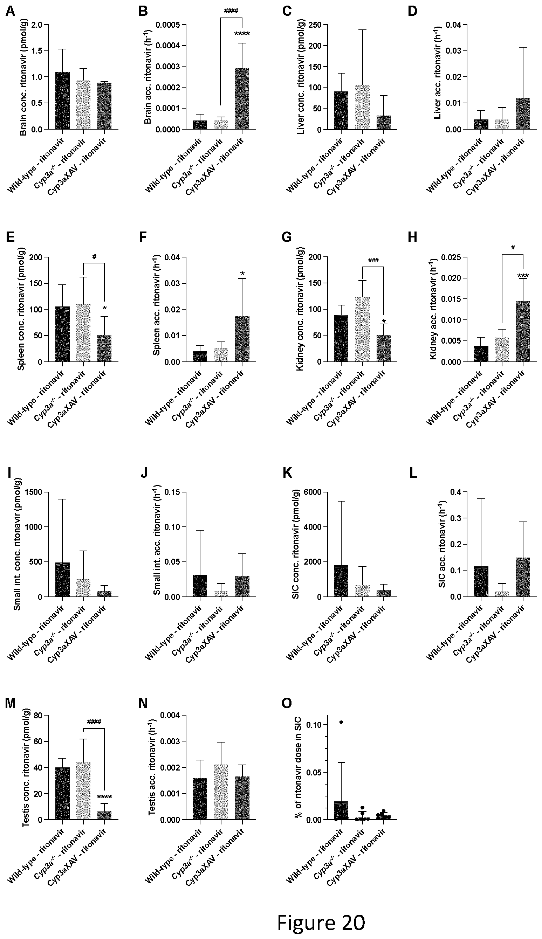

For the analyzed tissues at 24 hours, no meaningful differences were observed, except for the testis distribution. The testis disposition was significantly reduced in Cyp3aXAV mice compared to the other two strains (Fig. 20).

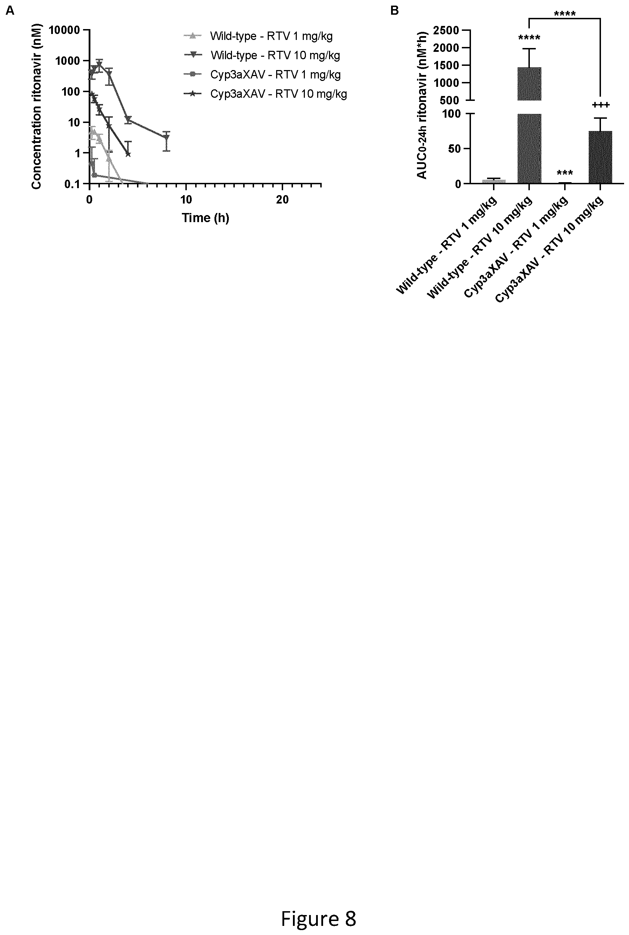

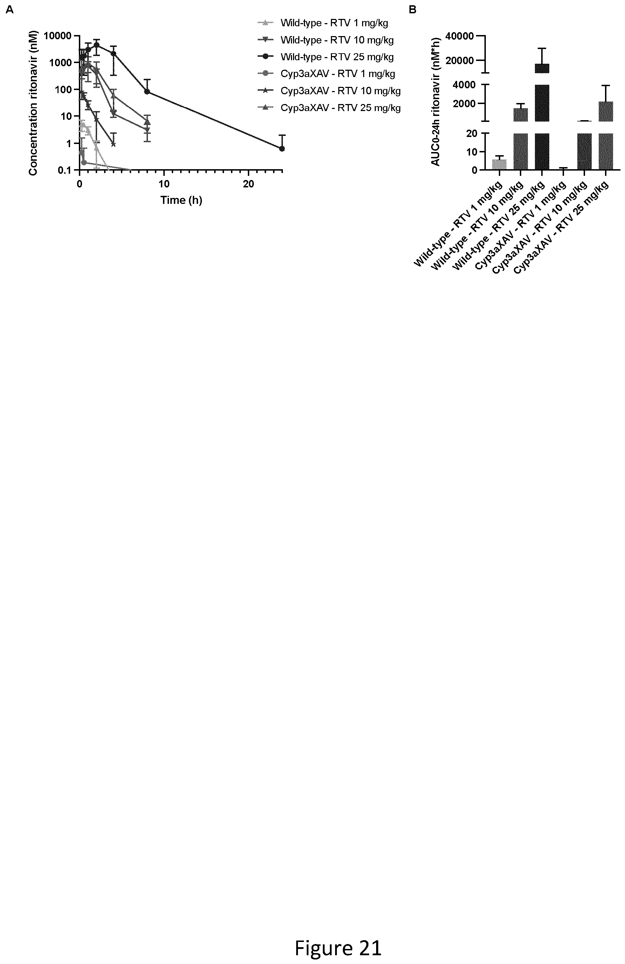

In the follow-up experiment with reduced ritonavir dosages, ritonavir tissue distribution was not assessed in view of the undetectable plasma concentrations at 24 hours. In this experiment differences in plasma exposure of ritonavir were more obvious. A clear, non-linear increase in the plasma $AUC_{0-24h}$ was observed in wild-type (244-fold, P < 0.0001) as well as Cyp3aXAV (168-fold, P < 0.0001) mice between the 1 mg/kg and 10 mg/kg groups (Fig. 8, Table 6). Similar results were observed for the Cmax, which was 130-fold increased between the wild-type groups, and 159-fold between the Cyp3aXAV groups (Fig. 8, Table 6). At the last time point (24 h) no ritonavir was detectable in any of the four experimental groups. It appears that the clearance of ritonavir itself is faster than that of cabazitaxel when comparing doses of 10 mg/kg for each drug. In Figure 21 the pharmacokinetic parameters of ritonavir of all three doses are combined, demonstrating the strong non-linearity in ritonavir plasma exposure between the different doses in both mouse strains.

**Table 5:** Pharmacokinetic parameters of ritonavir in male wild-type, Cyp3a-/-, and Cyp3aXAV mice over 24 hours after oral administration of 10 mg/kg cabazitaxel in combination with vehicle or 25 mg/kg ritonavir (n = 6). Data are presented as mean ± SD. $AUC_{0-24h}$, area under the plasma concentration-time curve from 0 to 24 hours; Cmax, maximum concentration in plasma; Tmax, time to reach maximum plasma concentration (h); Pbrain, brain accumulation per h; SIC, small intestinal content; SIC (% of dose), ritonavir in SIC as percentage of dose. ***, P < 0.001; ****, P < 0.0001 compared to wild-type pretreated with ritonavir. ##, P < 0.01; ###, P < 0.001 compared to Cyp3a-/- pretreated with ritonavir.

| Parameter | Type of pretreatment and genotype | | |
|---|---|---|---|
| | Ritonavir | | |
| | Wild-type | $Cyp3a^{-/-}$ | Cyp3aXAV |
| $AUC_{0-24h}$, nM.h | 17255 ± 12782 | 11749 ± 4660 | 2193 ± 1700****/### |
| Fold change $AUC_{0-24h}$ | 1.00 | 0.68 | 0.13 |
| $C_{max}$, nM | 4491 ± 2673 | 3010 ± 575 | 952 ± 785***/## |
| Fold change $C_{max}$ | 1.00 | 0.67 | 0.21 |
| $T_{max}$, h | 2.00 ± 0.00 | 2.33 ± 0.82 | 1.00 ± 0.55 |
| $C_{brain}$, pmol/g | 1.095 ± 0.438 | 0.952 ± 0.208 | 0.892 ± 0.018 |
| Fold change $C_{brain}$ | 1.00 | 0.87 | 0.81 |
| Brain-to-plasma ratio | 1.293 ± 0.781 | 1.107 ± 0.414 | 1.312 ± 0.027 |
| Fold change ratio | 1.00 | 0.86 | 1.01 |
| $P_{brain} \times 10^3$ (h$^{-1}$) | 0.043 ± 0.028 | 0.045 ± 0.013 | 0.283 ± 0.121****/#### |
| Fold increase $P_{brain} \times 10^3$ (h$^{-1}$) | 1.00 | 1.05 | 6.58 |
| $C_{Liver}$, pmol/g | 90.60 ± 43.32 | 107.4 ± 130.4 | 32.86 ± 47.62 |
| Fold change $C_{liver}$ | 1.00 | 1.19 | 0.36 |
| Liver-to-plasma ratio | 99.68 ± 59.56 | 91.88 ± 125.4 | 48.34 ± 70.07 |
| Fold change ratio | 1.00 | 0.92 | 0.48 |
| SIC (% of dose) | 0.019 ± 0.041 | 0.004 ± 0.005 | 0.005 ± 0.003 |
| Fold change | 1.00 | 0.21 | 0.26 |

**Table 6:** Pharmacokinetic parameters of ritonavir in male wild-type and Cyp3aXAV mice over 24 hours after oral administration of 10 mg/kg cabazitaxel in combination with vehicle, 1 mg/kg ritonavir or 10 mg/kg ritonavir (n = 6). Data are presented as mean ± SD. $AUC_{0-24h}$, area under the plasma concentration-time curve from 0 to 24 hours; Cmax, maximum concentration in plasma; Tmax, time to reach maximum plasma concentration (h). **, P < 0.01; ***, P < 0.001; ****, P < 0.0001 compared to wild-type pretreated with ritonavir 1 mg/kg. ++++, P < 0.0001 compared to Cyp3aXAV pretreated with ritonavir 1 mg/kg. &&&&, P < 0.0001 compared to wild-type pretreated with ritonavir 10 mg/kg.

| Parameter | Type of pretreatment and genotype | | | |
|---|---|---|---|---|
| | Ritonavir 1 mg/kg | | Ritonavir 10 mg/kg | |
| | Wild-type | Cyp3aXAV | Wild-type | Cyp3aXAV |
| $AUC_{0-24h}$, nM.h | 5.91 ± 1.80 | 0.447 ± 0.766*** | 1440 ± 529**** | 75.0 ± 18.8++++/&&&& |
| Fold change $AUC_{0-24h}$ | 1.00 | 0.076 | 243.7 | 12.7 |
| $C_{max}$, nM | 5.76 ± 1.38 | 0.532 ± 1.08** | 749 ± 322**** | 82.9 ± 15.9++++/&&&& |
| Fold change $C_{max}$ | 1.00 | 0.092 | 130.0 | 14.4 |
| $T_{max}$, h | 0.33 ± 0.13 | 1.38 ± 3.25 | 0.92 ± 0.20 | 0.25 ± 0.00 |

Example 4 - Development and validation of preclinical PK model

*Data analysis and software*

Population PK modeling was performed using nonlinear mixed-effects modeling in NONMEM (version 7.5.0, ICON Development Solutions, Ellicott City, MD, USA) with a gfortran compiler and Perl-speaks-NONMEM (version 5.3.0) (Beal SL SL. NONMEM user guides. Ellicott City, Maryland, USA. 1989; Lindbom et al. Comput Methods Programs Biomed. 2005). All models were fit with first order conditional estimation method with $\eta$-$\varepsilon$ interaction (FOCE-I). Pirana (version 2.9.9) was used as a modeling interface. R (version 4.0.4) was used for data management, visual model evaluation, and analysis and visualization of simulation data.

*PK data*

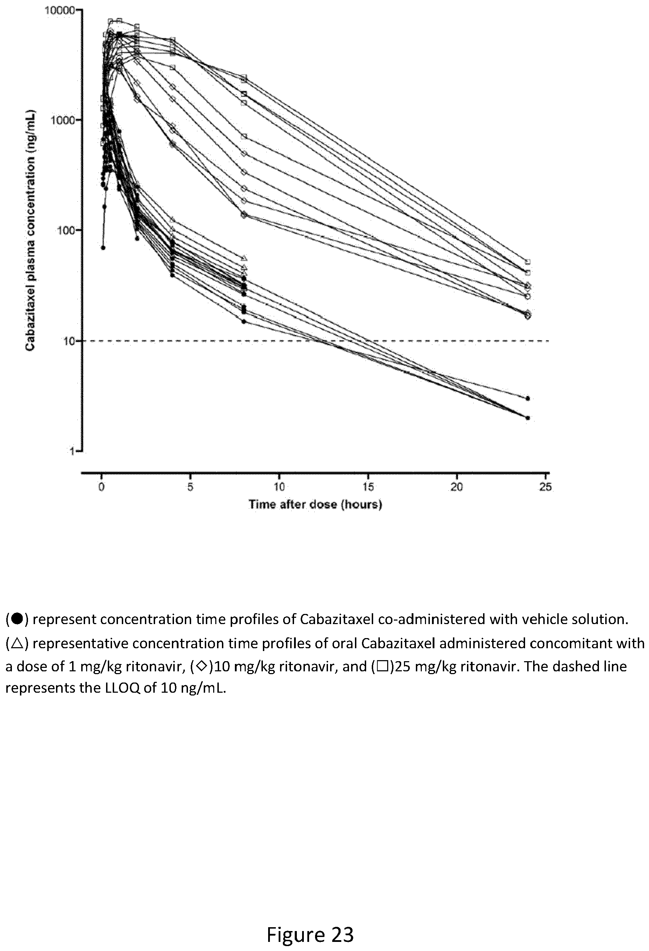

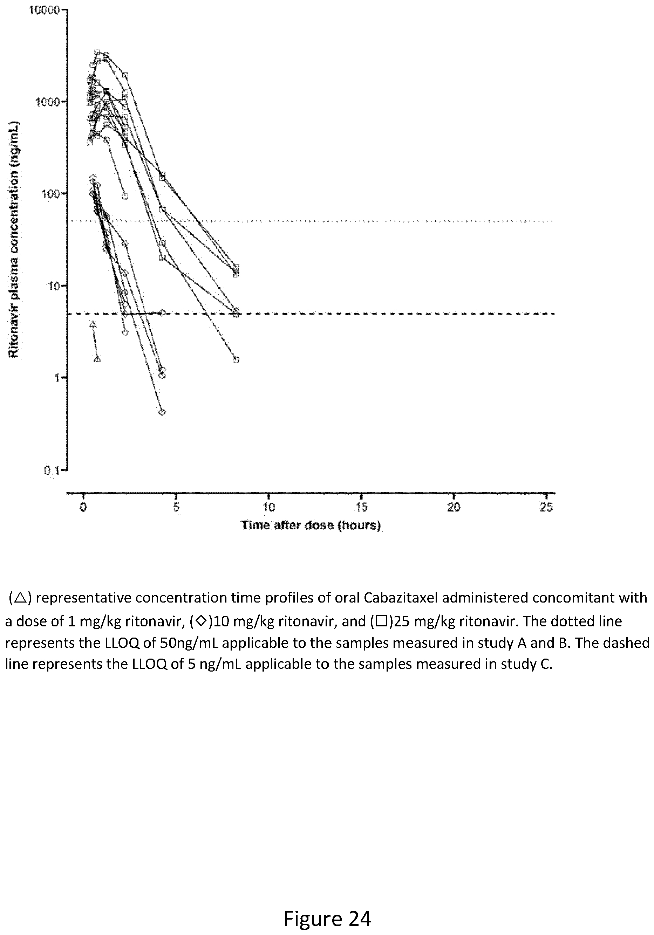

This analysis was performed using cabazitaxel PK data obtained in three preclinical studies in which the effect of several boosting drugs on the PK and tissue distribution of orally administered cabazitaxel was investigated. These studies included various transgenic and knockout mouse strains, including the Cyp3aXAV strain and are described in detail in Examples 1 to 3. Due to its predictive value for human hepatic CYP3A metabolism, the PK data obtained in the Cyp3aXAV mouse model was used in the current study (Damoiseaux et al. Pharmaceuticals. 2022). The characteristics of the included data are shown in Table 7. In total, cabazitaxel PK profiles were available from 42 Cyp3aXAV mice and were included in the current study. All mice received cabazitaxel (Jevtana®, Sanofi-Aventis groupe, Paris, France) by oral gavage at a dose of 10 mg/kg. In addition, 18 mice received cabazitaxel concomitant with vehicle solution (Polysorbate 80:ethanol:water, 1:1:4, (v/v)), 12 mice received oral cabazitaxel concomitant with ritonavir (Duchefa Farma, Haarlem, The Netherlands) at a dose of 25 mg/kg, 6 mice received oral cabazitaxel concomitant with ritonavir at a dose of 10 mg/kg, and 6 mice received oral cabazitaxel concomitant with ritonavir at a dose of 1 mg/kg. Ritonavir or vehicle solution were administered to the mice 15 minutes prior to oral cabazitaxel administration and all mice were in a fasted state before the first drug administration. Samples for determination of cabazitaxel and ritonavir plasma concentrations were taken from the mice tail vein at the following time points after administration: 0.25, 0.5, 1, 2, 4, 8, hours or 0.083, 0.167, 0.25, 0.5, 1 hours. Depending on the study, each experiment was terminated by cardiac puncture at either 2 hours or 24 hours after administration. Cabazitaxel and ritonavir concentrations in mouse plasma were measured using a validated liquid chromatography-tandem mass spectrometry (LC-MS/MS) method with a lower limit of quantification (LLOQ) of 10 ng/mL for cabazitaxel and 50 ng/mL (Study A, B) or 5 ng/mL (Study C) for ritonavir. The observed cabazitaxel and ritonavir plasma concentrations versus their corresponding time after dose are shown in Figures 23 and 24, respectively.

**Table** 7: Characteristics of preclinical studies included in the modeling and simulation study.

| Study | Mouse strain | Cabazitaxel dose (mg/kg) | Ritonavir dose (mg/kg) | PK sampling times (h) | No of mice | Weight (g, median (range)) |
|---|---|---|---|---|---|---|
| Total | Cyp3aXAV | 10 | | | 42 | 36.5 (30.6-42.3) |
| Study A | | | n.a. | 0.25, 0.5, 1, 2, 4, 8, 24 | 6 | 37.4 (32.0 - 40.1) |
| | | | 25 | | 6 | 37.0 (32.8 - 38.8) |
| Study B | | | n.a. | 0.083, 0.1, 0.25, 0.5, 1, 2 | 6 | 37.5 (34.5 - 40.2) |
| | | | 25 | | 6 | 35.4 (30.6 - 39.1) |
| Study C | | | n.a. | 0.25, 0.5, 1, 2, 4, 8, 24 | 6 | 36.2 (33.6 - 38.2) |
| | | | 1 | | 6 | 35.4 (32.4 - 42.3) |
| | | | 10 | | 6 | 35.6 (32.0 - 37.6) |

*Development of the preclinical PK model*

Ritonavir and cabazitaxel PK were modelled sequentially according to the population PK parameters and data (PPP&D) approach described by Zhang et al. (Zhang et al. J Pharmacokinet Pharmacodyn. 2003). In the development of the compartmental PK models, inclusion of model elements, such as the number of compartments, inter-individual variability (IIV), covariate effects, and residual random effects were guided by differences in objective function value (OFV), plausibility of parameter estimates, observation of trends in goodness of fit plots, and differences in estimation precision.

Cabazitaxel clearance was modelled using a well-stirred liver model, equivalent to what has previously been used to model the population PK of oral paclitaxel and docetaxel co-administered with ritonavir in humans (Yu et al. J Clin Pharmacol. 2020; Zhang et al. J Pharmacokinet Pharmacodyn. 2003; Wilkinson et al. Clin Pharmacol Ther. 1975). This well-stirred liver model assumes the liver to have a fixed volume and blood flow and hepatic clearance (CL) is calculated as a function of hepatic blood flow ($Q_1$) and intrinsic clearance ($CL_i$) following Eq.1.

$$CL = Q_1 \cdot \frac{CL_i}{CL_i + Q_1} \qquad \text{(Eq. 1)}$$

The effect of ritonavir on the relative gut bioavailability of cabazitaxel was modelled as a fixed effect (Eq.2). This estimate reflects the relative change in bioavailability for mice who received oral cabazitaxel concomitant with ritonavir versus mice who received cabazitaxel with vehicle. The ritonavir covariate effect on intrinsic cabazitaxel clearance was investigated both as a fixed effect, describing the relative change in cabazitaxel intrinsic clearance, and as a concentration-dependent effect following Eq.3.

$$rF_{gut} = 1 \cdot \theta_F^{RTV_i} , where\ RTV_i = 0\ or\ 1 \qquad \text{(Eq. 2)}$$

$$CL_i = \frac{CL_{i,0}}{1 + \frac{[RTV]}{K_i}} \qquad \text{(Eq. 3)}$$

Allometry was used to scale compartment volume ($V_H$, $V_1$, and V2) and clearance parameters ($CL_i$, $Q_1$, $Q_2$) to values for a 70 kg human with power coefficients of 1 and 0.75 for volume and clearance parameters, respectively. Thus, as an example, Eq. 4 gives the parameter estimate for the volume of distribution of the central compartment.

$$V_1 = V_{1\,(70)} \cdot \left(\frac{Weight}{70}\right)^1 \qquad \text{(Eq. 4)}$$

Which includes the weight of each individual mouse in kilograms, $V_1$ the volume of distribution and $V_{1(70)}$ the estimated volume of distribution for a 70 kg human.

In the variability model, it was assumed that IIV is log-normally distributed. With Eq. 5 defining the expression for the individual parameter estimate $\theta_i$.

$$\theta_i = \theta_{pop} \cdot e^{\eta_i} \qquad \text{(Eq. 5)}$$

Where $\theta_{pop}$ represents population mean parameter estimate and $\eta_i$ represents its individual deviation, which is distributed following $N\ (0,\ \omega^2)$. For both PK models, in was assumed that the residual error has both an additive and proportional component. The additive component of the error model was included only for observations which were designated as <LLOQ. Time points for which no quantity of either drug could be detected were discarded. For determination of the LLOQ a signal-to-noise ratio (S/N) of 10 is applied. Thus, a fixed additive error was set for observations <LLOQ which was set to 1/10th of the LLOQ for the respective compound. Consequently, individual model predictions are related to observations following Eq. 6.

$$C_{obs,ij} = C_{pred,ij} \cdot \left(1 + \varepsilon_{p,ij}\right) + (\varepsilon_{a,ij} \cdot LLOQ_{ij}),\ where\ LLOQ_{ij} = 0\ or\ 1 \qquad \text{(Eq. 6)}$$

Where $C_{obs,ij}$ represents the observation for the $i$th subject and the $j$th measurement. With $C_{pred,ij}$ the respective prediction. Both the proportional error $\varepsilon_{p,ij}$ and additive error $\varepsilon_{a,ij}$ were assumed to be distributed following $N\ (0,\ \sigma^2)$. $LLOQ_{ij}$ was included as a data record, which was set to one for observations that were below the limit of quantification.

*Model evaluation*

Evaluation of candidate models was based on plausibility and precision of parameter estimates, observation of trends in goodness-of-fit plots, $\eta$ and $\varepsilon$-shrinkage, and decreases in OFV for which a change in OFV of 3.84, corresponding to $\alpha=0.05$, was considered to be significant for hierarchical models. All models were required to achieve successful minimization. The predictive performance of the model was evaluated by simulating 500 datasets using the candidate models from which the 5th, 50th, and 95th percentiles were calculated, along with their 95% confidence intervals, which were compared with the 5th,50th, and 95th of the observations using a visual predictive check (VPC). The uncertainty in the final parameter estimates was evaluated using a sampling importance-resampling (SIR) algorithm (Dosne et al. J Pharmacokinet Pharmacodyn. 2017).

*Results*

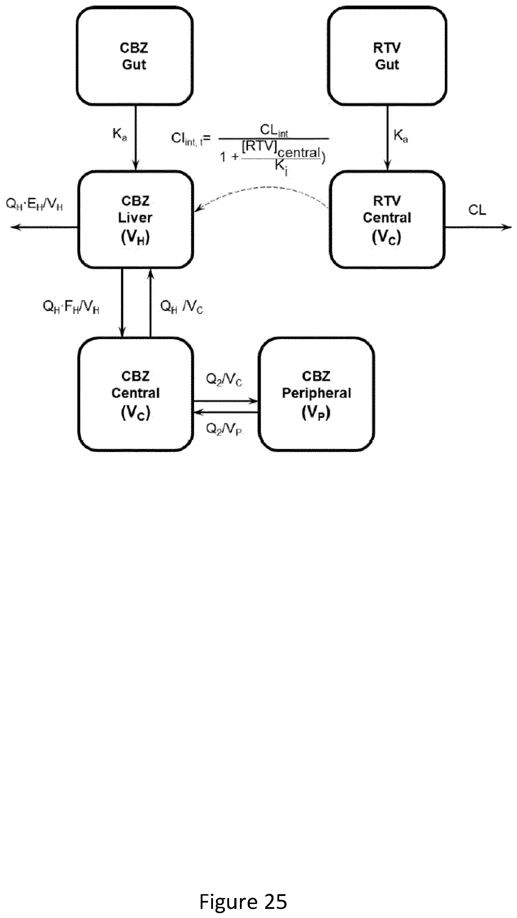

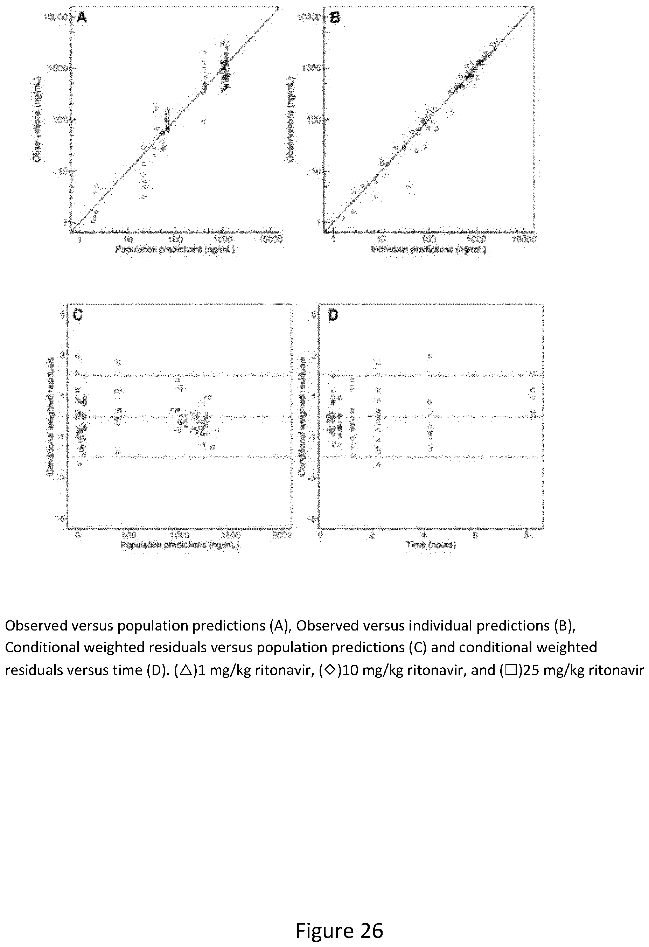

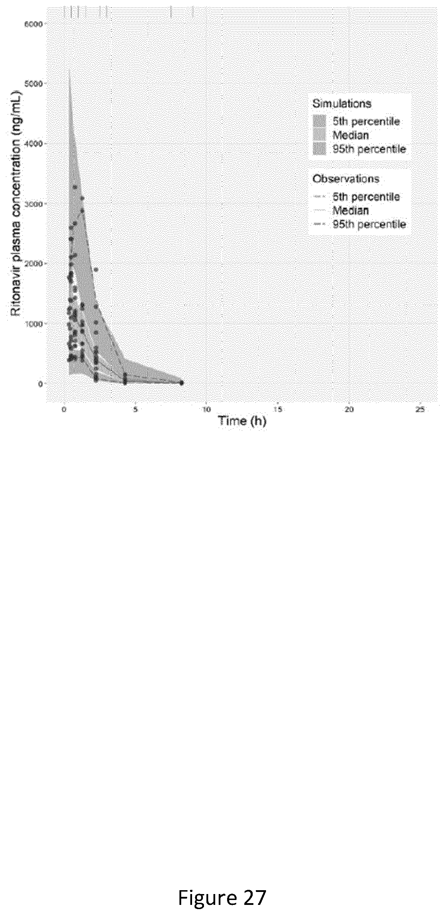

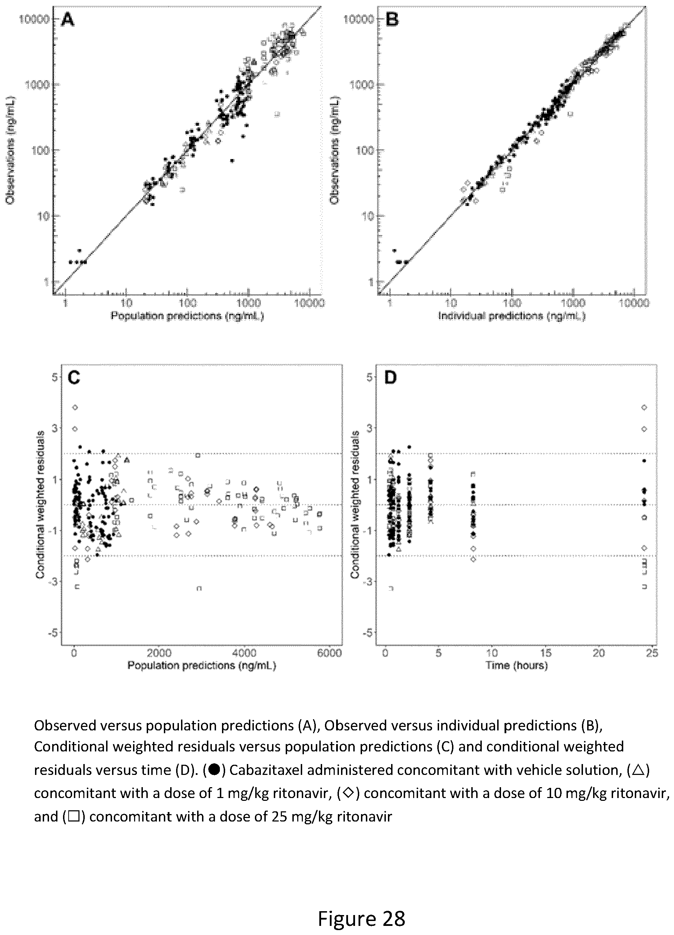

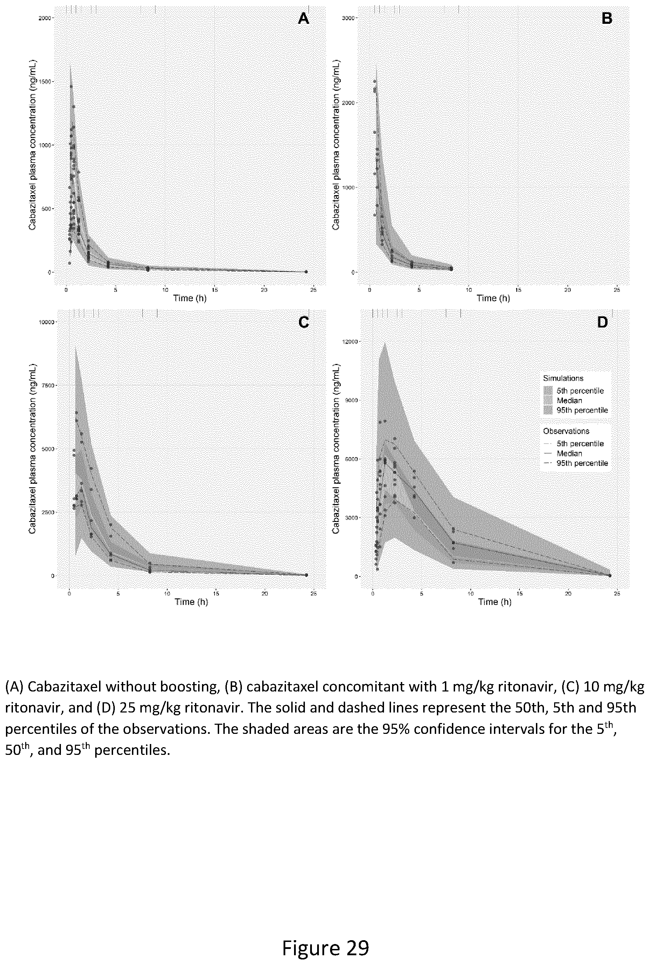

An overview of the structural model for orally administered cabazitaxel and ritonavir is provided in Figure 25. Ritonavir PK was modelled using a one-compartment model with first order absorption and linear elimination. Since ritonavir is known to inhibit its own CYP3A4 mediated metabolism, the final model included this effect on bioavailability for the different dose levels of ritonavir (Hsu et al. Clin Pharmacokinet. 1998). An effect of ritonavir dose on CL was investigated, but proved unidentifiable. Diagnostic plots (Figure 26) demonstrated no trends and, together with the results of the prediction-corrected VPC (Figure 27), showed that the developed model was suitable for the description of ritonavir PK in mice. The parameter estimates for the ritonavir model, allometrically scaled to a 70 kg human, are shown in Table 8.
The population PK of oral cabazitaxel in mice was best described using a two-compartment model with first order absorption. As intended, clearance and first-pass effect were modelled using a well-stirred liver model in which the effect of ritonavir on intrinsic cabazitaxel clearance was modelled as a concentration dependent covariate following a general competitive inhibition model. A mechanism-based inhibition model was investigated, but resulted in an increased OFV compared to the general competitive inhibition model. Since CYP2C8 also contributes to hepatic cabazitaxel metabolism, an inhibitory maximum effect (Imax) model was explored but parameters in this model proved unidentifiable. The inhibition constant Ki in the general competitive inhibition model was estimated to be 3.85 ng/mL, indicating a strong inhibitory effect of ritonavir plasma concentrations on intrinsic cabazitaxel clearance. Since the boosting effect of ritonavir on the gut bioavailability of cabazitaxel was assumed to be dose dependent, distinct effects were implemented for each ritonavir dose level. Similarly, fixed covariate effects were included for the decrease in first-order absorption rate as a result of the co-administration with 25 mg/kg and 10 mg/kg ritonavir (Hendrikx et al. Int J Cancer. 2013; Huisman et al. J Pharmacol Exp Ther. 2003). This covariate effect was not identifiable for mice receiving 1 mg/kg ritonavir.
The goodness of fit plots shown in Figure 28 demonstrate adequate model performance in predicting individual cabazitaxel concentrations when administered with vehicle and at every level of ritonavir boosting, while no trends in conditional weighted residuals versus time or population predictions were observed. The stratified VPC's (Figure 29) showed satisfactory predictive performance of the cabazitaxel PK model in all conditions. The final population PK parameter estimates allometrically scaled to a 70 kg human are shown in Table 8.

**Table 8:** Parameter estimates for the final population PK models for ritonavir and cabazitaxel in mice.

| Parameter (Unit) | Estimate | 95% CI[a] | Shrinkage (%) | Description |
|---|---|---|---|---|
| ***Ritonavir[b]*** | | | | |
| CL (L/h)[c] | 113 | 85.1 - 140 | - | Clearance |
| VC (L)[c] | 515 | 389 - 670 | - | Central volume |
| Ka (h[-1]) | 1.54 | 1.03 - 2.32 | - | First-order absorption rate constant |
| Frel 10 mg/kg | 0.14 | 0.09 - 0.21 | - | Relative gut bioavailability for 10 mg/kg dose |
| Frel 1 mg/kg | 0.04 | 0.01 - 0.11 | - | Relative gut bioavailability for 1 mg/kg dose |
| IIV Ka (CV%) | 68.9 | 46.9 - 0.95 | 25 | |

(continued)

| Parameter (Unit) | Estimate | 95% CI[a] | Shrinkage (%) | Description |
|---|---|---|---|---|
| ***Ritonavir[b]*** | | | | |
| IIV F (CV%) | 48.5 | 34.9 - 61.7 | 17 | |
| RUVprop (CV%) | 31.6 | 26.6 - 38.5 | 18 | |
| RUV add, <LLOQ,A,B (ng/mL) | 25 | Fixed | | |
| RUVadd,<LLOQ,C (ng/mL) | 0.25 | Fixed | | |
| ***Cabazitaxel*** | | | | |
| CLint (L/h)[c] | 106 | 95.1 - 117 | - | Uninhibited intrinsic clearance |
| VC (L)[c] | 293 | 264 - 329 | - | Central volume |
| Q2 (L/h)[c] | 27.7 | 23.9 - 32.1 | - | Inter compartment clearance |
| VP (L)[c] | 613 | 565 - 670 | - | Peripheral volume |
| QH (L/h)[c] | 80 | Fixed | - | Hepatic blood flow |
| VH (L)[c] | 1 | Fixed | - | Hepatic volume |
| Ka (h[-1]) | 5.83 | 4.15 - 8.11 | - | First-order absorption rate constant |
| Ki (ng/mL) | 3.85 | 2.20 - 7.23 | - | Inhibition constant for ritonavir on the intrinsic clearance of cabazitaxel |
| Frel 25 mg/kg | 3.65 | 3.06 - 4.38 | - | Relative gut bioavailability for 25 mg/kg dose |
| Frel 10 mg/kg | 2.42 | 1.79 - 3.25 | - | Relative gut bioavailability for 10 mg/kg dose |
| Frel 1 mg/kg | 1.13 | 0.87 - 1.47 | - | Relative gut bioavailability for 1 mg/kg dose |
| Ka, 25 mg/kg (h[-1]) | 1.22 | 0.76 - 2.03 | - | First-order absorption rate constant when co-administered with 25 mg/kg ritonavir |
| Ka, 10 mg/kg (h[-1]) | 2.62 | 1.28 - 5.32 | - | First-order absorption rate constant when co-administered with 10 mg/kg ritonavir |
| IIV Ka (CV%) | 63.4 | 49.2 - 85.1 | 15 | |
| IIV F (CV%) | 22.7 | 18.4 - 29.6 | 10 | |
| RUVprop (CV%) | 21.3 | 19.4 - 23.8 | 12 | |

(continued)

| Cabazitaxel | | | |
|---|---|---|---|
| RUVadd,<LLOQ (ng/mL) | 1 | Fixed | |

[a] 95% CI of parameter estimates computed with SIR on the final model

[b] Parameter estimates for ritonavir were fixed in the cabazitaxel PK model following the PPP&D approach (20).

[c] Parameter estimates allometrically scaled to a 70 kg human.

Example 5 - Extrapolation to human PK

A composite model was developed for the simulation of the PK of oral cabazitaxel in mCRPC patients. Parameter estimates for the structural and variability elements from a previously developed population PK model for IV cabazitaxel were used as the basis for the simulation of the PK of oral cabazitaxel in humans (Ferron et al. Cancer Chemother Pharmacol. 2013). This PK model was developed based on PK data obtained in 170 patients with advanced solid tumors treated with 1-hour infusions of 10-30 mg/m2 cabazitaxel in several Phase I-III trials. The IV model was expanded with a gut compartment and a well-stirred liver compartment to allow for simulation of ritonavir-boosted orally administered doses of cabazitaxel. The hepatic blood flow (QH) in the well-stirred liver model was fixed at 80 L/h (Leen et al. Gut. 1992). The volume of the hepatic compartment (VH) was set to 1 L, similar to previous models for oral taxanes (Yu et al. J Clin Pharmacol. 2020; Kan et al. J Nucl Med. 1979). The inhibition constant (Ki) for ritonavir on the intrinsic cabazitaxel clearance was the fundamental parameter translated from the preclinical PK model in mice to the composite human PK model. Since the Cyp3aXAV mouse model may prove a poor model for gut bioavailability for some CYP3A4 substrates (Damoiseaux et al. Pharmaceuticals. 2022), estimates for first-order gut absorption rate and gut bioavailability from previously published models were derived for oral docetaxel in humans (Yu et al. J Clin Pharmacol. 2020; Koolen et al. J Clin Pharmacol. 2012). Additionally the delay in gastric emptying time caused by ritonavir, resulting in an absorption lag time, was derived from a previously published model for indinavir in humans (Kappelhoff et al. Br J Clin Pharmacol. 2005; Hendrikx et al. Int J Cancer. 2013; Huisman et al. J Pharmacol Exp Ther. 2003). The PK of concomitantly administered oral ritonavir in humans was simulated with IIV using a previously developed population PK model (Yu et al. J Clin Pharmacol. 2020).

Monte Carlo simulations (n=1000) were performed using the composite model for oral cabazitaxel to simulate the PK. Dose levels of 1 through 40 mg were simulated for oral cabazitaxel. A dosing regimen of once daily cabazitaxel administered concomitant with 100 mg ritonavir, administered 15 minutes prior to cabazitaxel was assumed. The main dose limiting toxicity (DLT) of cabazitaxel is neutropenia (Mita et al. Clin Cancer Res. 2009; Diéras et al. Eur J Cancer. 2013), for which AUC has been shown to be a predictor (≥grade 3, $p<0.05$). Therefore, the selection of an equivalent dose level for oral cabazitaxel foremost was based on the attained equivalent AUC0-inf. Since threshold relationships have shown to predict both the efficacy and toxicity of other taxanes, equivalence to the IV cabazitaxel dose in terms of time above threshold plasma concentrations of 0.05 $\mu$M and 0.005 $\mu$M (tC>0.05, tC>0.005) was also evaluated (Joerger et al. Clin Cancer Res. 2007). PK of IV cabazitaxel following a dose of 25 mg/m2 was simulated for a typical patient of 1.9 m2. Linear interpolation of the 25th, 50th, and 75th percentiles of the simulated metrics per dose level was used to calculate an equivalent oral dose. A sensitivity analysis was performed by assessing the effect of 10-fold decrease and a 50-fold increase in the estimate for Ki on the final equivalent oral dose levels with respect to the various metrics.

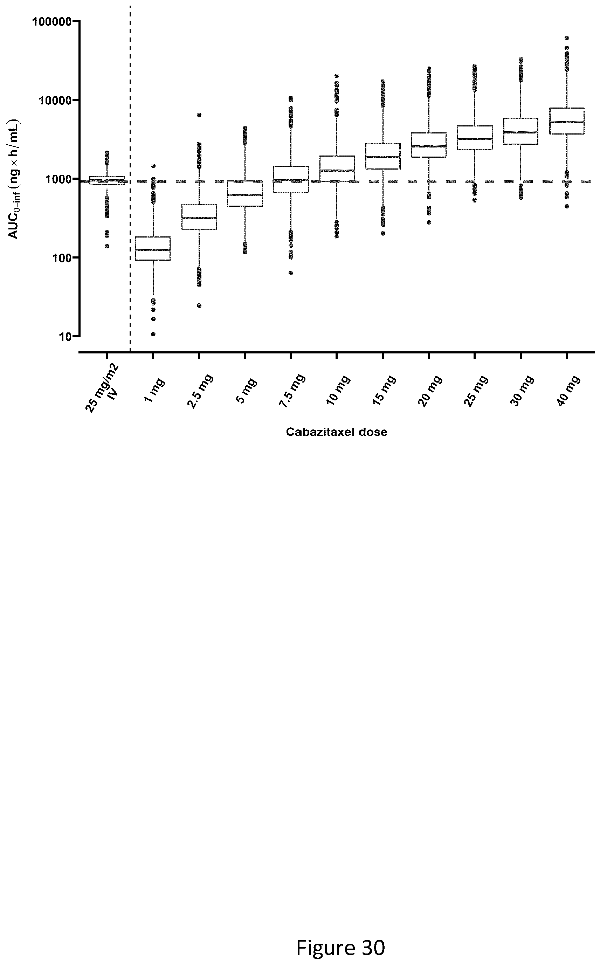

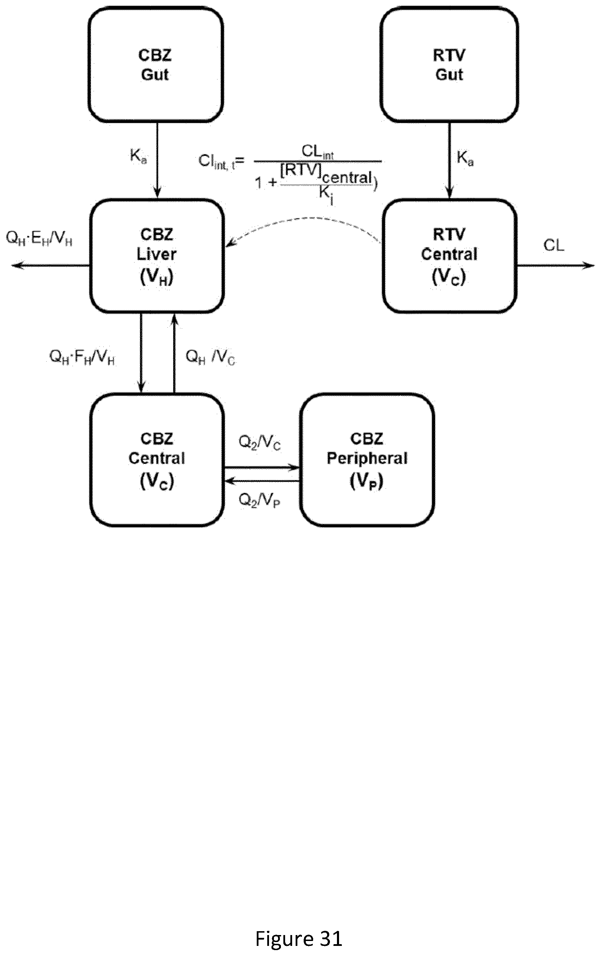

The preclinical Ki estimate was integrated in a published model for IV cabazitaxel (Ferron et al. Cancer Chemother Pharmacol. 2013), which was expanded with parameter estimates from population PK models for oral docetaxel, ritonavir, and indinavir (Yu et al. J Clin Pharmacol. 2020; Kappelhoff et al. Br J Clin Pharmacol. 2005). The complete composite model structure is shown in Figure 30. The corresponding input parameter values used to simulate the kinetics of oral cabazitaxel along with their source are shown in Table 9 below. This composite model was used with the preclinically informed Ki to simulate human exposure to oral cabazitaxel boosted with 100 mg ritonavir in terms of relevant exposure metrics. The results of calculations of AUC0-inf, tC>0.05 and tC>0.005 based on 1000 Monte Carlo simulations for each dose level with the composite model for oral cabazitaxel are shown in Figure 31 and Table 10. Simulation of tC>0.05 indicate a large proportion (>10%) of simulated patients at dose levels of 1 to 20 mg who do not achieve any time above the 0.05 $\mu$M threshold (data not shown). The simulated median AUC0-inf, tC>0.05 and tC>0.005 for IV cabazitaxel dosed at 25 mg/m2 for a typical 1.9 m2 patient were 921 ng·h/mL, 1.17 hours and 12 hours, respectively. Oral doses equivalent to IV dose in terms of the three PK metrics was calculated using linear interpolation of the 25th, 50th and 75th percentiles of the respective metrics at each dose level simulated with the oral cabazitaxel model (Table 11). The median AUC0-inf achieved after administration of an oral dose of 7.4 mg (inter quartile range (IQR): 5.2 mg) boosted with 100 mg ritonavir was equivalent to the registered IV dose. Equivalence in terms of tC>0.05 and tC>0.005 for a median patient was achieved at boosted oral doses of 6.4 (IQR: 12.1) and 5.3 (IQR: 9.8), respectively.

**Table 9:** Input parameter values used to simulate the pharmacokinetics of oral cabazitaxel in humans (References: 17: Ferron et al. Cancer Chemother Pharmacol. 2013; 18: Yu et al. J Clin Pharmacol. 2020; 26: Koolen et al. J Clin Pharmacol. 2012; 27: Kappelhoff et al. Br J Clin Pharmacol. 2005)).

| Parameter in Simulation | Estimate | Source | Comments |
|---|---|---|---|
| **Cabazitaxel** | | | |
| $CL_{int}$ (L/h) | 123.17 | (17,18) | Intrinsic clearance calculated following $$Cl_H = Q_H \cdot \frac{Cl_{int}}{Q_H + Cl_{int}}$$ With $Cl_H$ 48.5 L/h and $Q_H$, 80 L/h |
| V1(L) | 26 | (17) | - |
| K12 ($h^{-1}$) | 2.48 | (17) | - |
| K21 ($h^{-1}$) | 0.604 | (17) | - |
| K13 ($h^{-1}$) | 4.84 | (17) | - |
| K31 ($h^{-1}$) | 0.0266 | (17) | - |
| $O_H$ (L/h) | 80 | (18) | |
| $V_H$ (L) | 1 | (18) | - |
| Ka ($h^{-1}$) | 0.7 | (26) | First order absorption rate constant reported for docetaxel |
| $F_{rel, gut}$ | 3.66 | (18) | Effect of concomitant treatment with ritonavir on the relative gut bioavailability of docetaxel |
| Lag-time on Ka (h) | 0.483 | (27) | Lag-time observed for indinavir PK when co-administered with ritonavir |
| $K_i$ (ng/mL) | 3.85 | Preclinical cabazitaxel PK | Inhibition constant of ritonavir on intrinsic cabazitaxel clearance |
| IIV CL (CV%) | 38.8 | (17) | - |
| IIV V1 (CV%) | 93.4 | (17) | - |
| IIV K12 (CV%) | 84.0 | (17) | - |
| IIV K13 (CV%) | 64.2 | (17) | - |
| IIV K31 (CV%) | 28.2 | (17) | - |
| IOV CL (CV%) | 19,4 | (17) | - |
| IOV V1 (CV%) | 45.3 | (17) | - |
| **Ritonavir** | | | |
| MAT (h) | 8.45 | (18) | - |
| CV (%) | 123 | (18) | - |
| CL (L/h) | 7.72 | (18) | - |
| V (L) | 23 | (18) | - |
| Q (L/h) | 3.99 | (18) | - |
| Vp (L) | 17.9 | (18) | - |
| F | 1.06 | (18) | - |

(continued)

| Ritonavir | | | |
|---|---|---|---|
| IIV CV (CV%) | 12.8 | (18) | - |
| IIV CL (CV%) | 46.7 | (18) | - |
| IIV Vc (CV%) | 93.5 | (18) | - |
| IIV F (CV%) | 52.2 | (18) | - |
| IIV $F_{tatler}$(CV%) | 30.0 | (18) | - |
| IOV MAT (CV%) | 32.1 | (18) | - |
| IOV CV (CV%) | 22.2 | (18) | - |

**Table** 10: $T_{c>0.05}$ and $t_{c>0.005}$ calculated based on Monte Carlo simulations (n=1000) of IV cabazitaxel (25 mg/m$^2$) simulated for a typical patient with a BSA of 1.9 m$^2$ and oral cabazitaxel at dose levels 1 - 40 mg concomitant with 100 mg ritonavir

| | $t_{c>0.05}$ (h) | | | $t_{c>0.005}$ (h) | | |
|---|---|---|---|---|---|---|
| Cabazitaxel dose | Median | 25th percentile | 75th percentile | Median | 25th percentile | 75th percentile |
| IV 25 mg/m$^2$ | 1.18 | 1.04 | 1.44 | 13.5 | 6.27 | 37.6 |
| Oral 1 mg | 0.00 | 0.00 | 0.00 | 2.32 | 0.00 | 4.99 |
| Oral 2.5 mg | 0.00 | 0.00 | 0.67 | 5.88 | 2.63 | 13.3 |
| Oral 5 mg | 0.00 | 0.00 | 2.67 | 10.4 | 4.91 | 50.5 |
| Oral 7.5 mg | 1.53 | 0.00 | 3.91 | 37.3 | 6.78 | 78.0 |
| Oral 10 mg | 2.38 | 0.00 | 5.37 | 66.9 | 8.86 | 97.8 |
| Oral 15 mg | 3.38 | 1.14 | 6.82 | 104 | 19.2 | 137 |
| Oral 20 mg | 4.72 | 2.22 | 9.13 | 144 | 100 | 172 |
| Oral 25 mg | 5.59 | 2.77 | 12.7 | 167 | 126 | 199 |
| Oral 30 mg | 6.29 | 3.27 | 21.5 | 193 | 145 | 232 |
| Oral 40 mg | 8.89 | 4.19 | 38.8 | 230 | 175 | 280 |

A sensitivity analysis was performed by applying a 10-fold decrease and a 50-fold increase in the estimate of Ki (Table 11). Relatively little effect was observed of a decreased Ki on the estimated equivalent dose for all evaluated PK metrics. Conversely, a profound increase in Ki resulted in a ~2-fold increase in the equivalent oral cabazitaxel dose for all metrics.

**Table 11:** Dose levels of oral cabazitaxel achieving similar exposure to the registered IV cabazitaxel dose of 25 mg/m$^2$ in terms of AUC$_{0\text{-inf}}$ (ng × h/mL), t$_{C>0.05}$ (h), t$_{C>0.005}$ (h) calculated using the preclinical K$_i$ estimate, a 50-fold increased K$_i$ estimate, and a 10-fold decreased K$_i$ estimate

| PK Metric | Predicted equivalent dose (mg) Based on preclinical K$_i$ estimate | | | Predicted equivalent dose (mg) 50-fold increase in K$_i$ estimate | | | Predicted equivalent dose (mg) 10-fold decrease in K$_i$ estimate | | |
|---|---|---|---|---|---|---|---|---|---|
| | Median | 25th percentile | 75th percentile | Median | 25th percentile | 75th percentile | Median | 25th percentile | 75th percentile |
| AUC$_{0\text{-inf}}$ | 7.4 | 10.3 | 5.1 | 16.4 | 23.4 | 12.1 | 6.0 | 8.7 | 3.7 |
| T$_{C>0.05}$ | 6.4 | 15.2 | 3.1 | 12.0 | 23.7 | 6.2 | 6.3 | 14.4 | 2.7 |
| T$_{C>0.005}$ | 5.3 | 12.3 | 2.5 | 15.2 | 26.2 | 8.3 | 5.0 | 10.5 | 1.6 |

Conclusion

The Examples 1 to 3 demonstrated that orally administered ritonavir can dramatically enhance the oral availability and tissue distribution of cabazitaxel by inhibition of mouse Cyp3a and human CYP3A4. This is consistent with the described metabolism of cabazitaxel, which is highly dependent on CYP3A4/5 and to a lesser extent on CYP2C8 in humans (FDA, U.S.F.a.D.A., Prescribing information Jevtana (cabazitaxel). 2010; EMA, Assessment Report for Jevtana (cabazitaxel). 2011). It was further demonstrated that also reduced doses of ritonavir can still exhibit substantial inhibitory effects on Cyp3a and CYP3A4 and increase the oral availability of cabazitaxel. Interestingly, a strong non-linearity between the 1 mg/kg, 10 mg/kg and 25 mg/kg ritonavir doses in the transgenic CYP3A4 mouse strain was observed, which could be of clinical relevance. Comparing the obtained data of Cyp3a deficient mice with a previously performed study by Tang et al. (2015), the data showed an almost 17-fold enhancement of the plasma $AUC_{0-24h}$ in Cyp3a-/- compared to wild-type mice, in contrast to the previously observed differences of approximately 6-fold (van Waterschoot et al. Cancer Res. 2009). It was observed that the plasma exposure to cabazitaxel of transgenic CYP3A4 mice was even lower than that in wild-type mice, both without ritonavir pretreatment. In addition, the oral availability in both wild-type and Cyp3aXAV mice could be boosted by the coadministration of ritonavir, reaching plasma levels close to the concentrations observed in the Cyp3a-/- mice, at least during the absorption phase of the drug. Therefore, these results show that the oral availability of cabazitaxel is highly restricted by CYP3A activity and this could be strongly boosted by the use of the CYP3A inhibitor ritonavir. The results of these Examples are therefore a good starting point to test an oral cabazitaxel formulation in combination with ritonavir in a small patient population. Designing a suitable oral formulation of cabazitaxel may require careful considerations, but possibly a similar formulation as for oral paclitaxel and docetaxel could be used (Lin et al. Drug Metab Dispos. 2013; Jibodh et al. Eur J Pharmacol. 2013; Sawicki et al. Int J Pharm. 2016; Vermunt et al. Cancer Rep (Hoboken). 2021).

Ritonavir is an essentially irreversible inhibitor of CYP3A, which means that CYP3A remains non-functional after inactivation by ritonavir (Tang et al. Mol Pharm. 2015; Rock et al. Mol Pharmacol. 2014). The inhibited CYP3A will only be replaced by newly synthesized enzymes. This process can take maximally three days in humans based on the enterocyte turnover rate (Darwich et al. Drug Metab Dispos. 2014). The Cyp3a turnover rate in mice will likely be more rapid, because they are smaller organisms that tend to have a faster-running physiology. Probably, the high intestinal expression of human CYP3A4 in the Cyp3aXAV mice results in a relatively fast biotransformation of ritonavir and subsequently cabazitaxel in this mouse strain compared to wild-type mice, after replacement of the inactivated CYP3A4 in their liver and especially intestine. This hypothesis could also be derived from the current data in the Cyp3aXAV mice pre-treated with ritonavir compared to wild-type mice, in which the plasma levels of cabazitaxel as well as ritonavir were lower in the transgenic mouse strain.

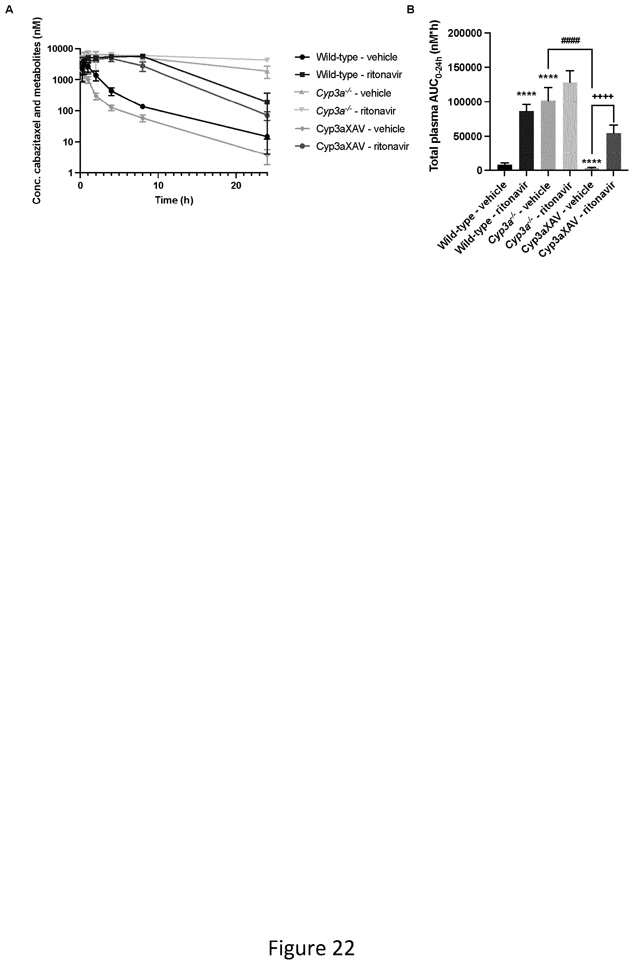

The Examples also demonstrated that ritonavir (25 mg/kg) has a strong impact on the formation of the active metabolites of cabazitaxel. Interestingly, the combined plasma exposure of the active compounds (cabazitaxel plus DM1, DM2, and docetaxel) does not show a substantially different plasma exposure profile in the different strains and conditions compared to the results seen for the main active component cabazitaxel (compare Fig. 1A and C and Fig. 22). In the ritonavir-pretreated groups the measured concentrations of the three metabolites were lower and their apparent formation peaked at a later time point compared to their vehicle groups, but cabazitaxel was more abundant compared to the metabolites. Similar, albeit quantitatively smaller shifts were obtained with the lower doses of ritonavir (1 mg/kg and 10 mg/kg, respectively), although the results for the 1 mg/kg ritonavir group were closer to those of the vehicle-treated Cyp3aXAV mice. This high impact on the metabolite formation was not unexpected, because cabazitaxel in humans is for more than 80% metabolized by CYP3A (FDA, U.S.F.a.D.A., Prescribing information Jevtana (cabazitaxel). 2010; EMA, Assessment Report for Jevtana (cabazitaxel). 2011). Interestingly, in the metabolite-to-parent drug ratios of the transgenic Cyp3aXAV mice pretreated with all different ritonavir doses (Fig. 12 and Fig. 19), some DM2 ratios of above 1 were obtained, which is higher than the described amounts of active metabolites in human plasma (FDA, U.S.F.a.D.A., Prescribing information Jevtana (cabazitaxel). 2010). Under normal circumstances, none of the three active metabolites should account for more than 10% of the total plasma exposure in human (FDA, U.S.F.a.D.A., Prescribing information Jevtana (cabazitaxel). 2010). This suggests that the mouse model possibly expresses higher levels of CYP3A4 in liver and/or intestine compared to humans (on average).

Besides the inhibitory effects of ritonavir on CYP3A, ritonavir is also a P-gp (ABCB1) inhibitor (Kharasch et al. Clin Pharmacol Ther. 2008). This efflux transporter plays an especially important role in the blood-brain-barrier (BBB), the liver and the intestine (Szakács et al. Drug Discov Today. 2008; Choudhuri et al. Int J Toxicol. 2006). However, the inhibitory potency of oral ritonavir for Abcb1 seems to be more pronounced in the liver and small intestine, and not in the BBB (Kharasch et al. Clin Pharmacol Ther. 2008; Holmstock et al. Drug Metab Dispos. 2010; Bachmeier et al. Pharm Res. 2005). This was also observed for the oral taxanes docetaxel and paclitaxel, in which the plasma exposure was enhanced after ritonavir coadministration, but not the relative brain accumulation of the drugs (de Weger et al. Clin Pharmacol Drug Dev. 2021). In the present Examples, the brain penetration of cabazitaxel with or without ritonavir was also studied, although brain metastases are rare in prostate cancer and the exact incidence is even unknown (McBean et al. J Clin Imaging Sci. 2021; Bhambhvani et al. World Neurosurg. 2020; Myint et al. Med Sci Monit. 2021). However, there are indications that patients suffering from visceral metastasis had a higher chance of brain metastasis in prostate cancer (Myint et al. Med Sci Monit. 2021 ). It was observed that the relative brain-to-plasma ratio and brain accumulation in wild-type and transgenic CYP3A4 mice was about 2-fold increased in the ritonavir pretreated group compared to the vehicle group (Fig. 2). This might indicate that ritonavir has some impact on increasing the brain disposition of cabazitaxel. However, since these comparisons were made under highly divergent plasma concentrations and exposures of cabazitaxel, these results should be interpreted with great caution. In Cyp3a-/- mice for instance, which had the highest plasma ritonavir exposure (Fig. 7) and a relatively limited difference in plasma cabazitaxel concentrations with or without ritonavir, no significant increase in brain-to-plasma ratio or brain accumulation of cabazitaxel was observed upon ritonavir coadministration (Fig. 3). It should be noted, though, that at the time of measurement (24 hours) the plasma ritonavir concentration had already gone down very considerably (Fig. 7). Altogether, whether the brain accumulation of cabazitaxel was indeed modestly increased by significant inhibition by ritonavir of P-gp/Abcb1 at the BBB therefore remains doubtful.

Furthermore, it was found that the effect of both mouse Cyp3a and human CYP3A4 on cabazitaxel metabolism could be partly or completely reversed with the coadministration of the CYP3A inhibitor ritonavir at different dose levels. The results of the Examples showed that the highest ritonavir dose (25 mg/kg) led to complete inhibition of the enzyme, whereas the other two tested doses (1 and 10 mg/kg) caused only partial inactivation. As disclosed above, two cases of transient early diarrhea were observed in two of the Cyp3a-/- mice pretreated with ritonavir at a dose of 25 mg/kg (total n = 6). This transient toxicity in this mouse strains may be related to the high plasma exposure to cabazitaxel at this dose level of ritonavir, because in none of the other strains this toxicity was observed, nor at the two lower ritonavir dose levels. Another possibility is that the observed diarrhea is related to ritonavir exposure itself, as this is a known side effect of ritonavir. Furthermore, this toxicity also shows that it is important to administer the lowest ritonavir dose possible to obtain just about sufficient CYP3A inhibition, when moving to a clinical application of oral cabazitaxel. By coadministering a lower ritonavir dose with cabazitaxel, side effects of ritonavir as well as cabazitaxel itself could be reduced to a minimal level. When converting this administered dose of 10 mg/kg for mice to the human equivalent dose, this would result in a dose of approximately 65 mg ritonavir per day (Nair et al. J Basic Clin Pharm. 2016). Interestingly, this calculated human equivalent dose is lower than the conventionally used ritonavir boosting doses (Eichbaum et al. Eur J Clin Pharmacol. 2013).

In Example 4, a population PK model of oral cabazitaxel was developed, co-administered with or without ritonavir, in 42 Cyp3aXAV mice and applied allometric scaling to scale relevant parameters to those for a 70 kg human patient. From this model, the critical parameter that quantifies the effect of ritonavir on hepatic intrinsic cabazitaxel clearance was extrapolated to a composite model for oral cabazitaxel to predict exposure in terms of AUCO-inf, tC>0.05 and tC>0.005 after administration of a range of ritonavir-boosted oral cabazitaxel doses in man.

Preclinical experiment results were used to quantify the inhibition of cabazitaxel hepatic metabolism by ritonavir. This Ki estimate of 3.85 ng/mL was relatively low, indicating a profound inhibitory effect of ritonavir on intrinsic hepatic cabazitaxel clearance. In comparison, a similar study by the group in which translational modeling of preclinical docetaxel PK to humans was performed using a similar model structure estimated Ki at 470 ng/mL (Koolen et al. J Clin Pharmacol. 2012). However, this model was developed based on data of oral docetaxel PK in wild type and several knockout (Mdr1a/1b-/-, Cyp3a-/-, and Cyp3a/Mdr1a/1b-/-) mouse models, while the current analysis only included data obtained from the Cyp3aXAV mouse model (Damoiseaux et al. Pharmaceuticals. 2022). Moreover, in the docetaxel model, no ritonavir data was available and ritonavir PK was allometrically scaled from humans to mice which may have resulted in an overpredicted Ki (Koolen et al. J Clin Pharmacol. 2012) . This was confirmed by the comparison of the model predicted PK with the results from a proof-of-concept study with ritonavir-boosted oral docetaxel which indicated a strong underprediction of human docetaxel plasma concentrations based on the Ki estimate (Koolen et al. J Clin Pharmacol. 2012) . Estimates for the ritonavir Ki in population PK models for oral docetaxel in humans of 210 ng/mL and 28 ng/mL have been reported (Yu et al. J Clin Pharmacol. 2020; Koolen et al. Br J Clin Pharmacol. 2010). The latter of which approximates the Ki estimate found in the preclinical study.

The composite simulation model for oral cabazitaxel in humans incorporated the Ki estimate derived from the mice experiments and was used to simulate several exposure metrics for oral cabazitaxel in mCRPC patients. The structural and variability parameter estimates for a previously developed model for IV cabazitaxel were expanded with structural elements from other models to allow for the simulation of the PK of different doses of orally administered cabazitaxel. A previous study has shown that mCRPC patients have a both a decreased systemic exposure to docetaxel and a lower risk of experiencing severe neutropenia (De Vries Schultink et al. Cancer Med. 2019) . The exact mechanism for this difference in exposure remains to be elucidated. Nevertheless, the population PK model for IV cabazitaxel used in the simulations accounts for differences in exposure between patients with breast cancer and various other tumor types (mainly prostate cancer) by estimating a covariate effect of breast cancer tumor type on clearance (Ferron et al. Cancer Chemother Pharmacol. 2013). By implementing this model without the covariate effect in the simulations an increased clearance of cabazitaxel in the simulations was accounted for. Unfortunately, the data available did not allow for the quantification of potential differences in the inhibition of cabazitaxel clearance as a result of ritonavir co-administration. The fixed effect for the increase in cabazitaxel gut bioavailability when co-administered with ritonavir, was derived from a previous model for oral docetaxel. Since human gut bioavailability may be sub optimally predicted by the Cyp3aXAV mouse model (Damoiseaux et al. Pharmaceuticals. 2022). The first-order absorption rate constant was based on the assumption that 50% of the bioavailable dose would be absorbed in 1 hour and the effect of ritonavir on gastric emptying time was derived from a population PK model for indinavir co-administered with ritonavir (Koolen et al. J Clin Pharmacol. 2012; Kappelhoff et al. Br J Clin Pharmacol. 2005). Since AUC0-inf is not influenced by the rate of absorption or lag time, the additional uncertainty as a result of these assumed or extrapolated parameters is not expected to influence the final estimate for an equivalent dose. Time above threshold metrics on the other hand may be influenced by the parameter estimate for absorption rate although this effect is considered to be limited. Overall, the assumptions made in the simulations may be validated by the empirical PK results obtained in first-in-human studies with oral cabazitaxel.

The modeling and simulation setup did not account for the role of other CYP enzymes in the metabolism of cabazitaxel. While CYP3A enzymes are largely (80 - 90%) responsible for cabazitaxel metabolism, a lesser fraction is also metabolized by CYP2C8 (Sanofi-Aventis groupe. Summary of Product Characteristics: Jevtana, INN-cabazitaxel. 2011). Such other metabolic pathways may become more pronounced in the presence of strong CYP3A4 inhibition by ritonavir. In addition, ritonavir may induce CYP2C8 (AbbVie BV. Norvir, INN-ritonavir-Summary of Product Characteristics. 2019). Both processes may increase biotransformation of cabazitaxel and result in a decreased systemic exposure. Another factor to consider when comparing the PK of oral and IV cabazitaxel are dissimilarities in the formulations. Previous oral taxane formulations have been developed as amorphous solid dispersions, the excipients from which show little to no bioavailability or effect on systemic drug disposition (Moes et al. Eur J Pharm Biopharm. 2013; Moes et al. Int J Pharm. 2011). In contrast, the registered IV cabazitaxel formulation contains polysorbate 80, which has been reported to increase the fraction of unbound docetaxel (Sanofi-Aventis groupe. Summary of Product Characteristics: Jevtana, INN-cabazitaxel. 2011; Loos et al. Clin Pharmacol Ther. 2003). However, polysorbate 80 is quickly eliminated in humans, which may indicate that this will not have a relevant effect in the comparison between oral and IV administration.

The main goal of phase I trials is to establish a safe dose of an experimental drug in patients (Le Tourneau et al. J Natl Cancer Inst. 2009). For cytotoxic agents, the recommended phase 2 dose is often a dose level close to the maximum tolerated dose (MTD), balancing toxicity and efficacy of treatment. The MTD is determined through sequentially exposing cohorts of patients to increasing doses following predefined dose-escalation methods with toxicity as the primary endpoint. The traditional and commonly used rule-based 3+3 design proceeds with cohorts of 3 patients treated at increasing doses, in which dose escalation frequently follows a modified Fibonacci scheme (Le Tourneau et al. J Natl Cancer Inst. 2009). The starting dose is often, following ICH S9 recommendations, a dose which is 1/10th of the dose causing severe toxicity in 10% of animals, which may be scaled to humans according to BSA, body weight, AUC, or other exposure parameters (International Council for Harmonisation of Technical Requirements for Pharmaceuticals for Human Use. ICH S9 - Non-Clinical evaluation for anti-Cancer pharmaceuticals. Gl. 2013). As a result, such designs may require inclusion of multiple cohorts and potentially expose many patients to subtherapeutic doses.

Example 5 demonstrated that an oral cabazitaxel dose of 7.4, 6.3, or 5.4 mg in combination with 100 mg ritonavir yielded equivalent exposure to the approved IV dose of 25 mg/m2 in an 1.9 m2 patient in terms of AUCO-inf, tC>0.05, and tC>0.005, respectively. Since AUC0-48h has shown to correlate well with a decrease in neutrophil count in phase I studies with IV cabazitaxel, the dose calculated based on the AUC0-inf exposure metric may be considered most appropriate for implementation in phase I study design (Mita et al. Clin Cancer Res. 2009; Diéras et al. Eur J Cancer. 2013) This equivalent oral dose of 7.4 mg may be used in a traditional 3+3 design to better define the range of dose levels to be investigated or to allow for more rigorous dose-escalation. Other rule-based designs, such as an accelerated titration (ATD) design (Simon et al. J Natl Cancer Inst. 1997) or an Bayesian Optimal Interval (BOIN) design (Yuan et al. Clin Cancer Res. 2016), may be exceptionally suited to incorporate a prior estimate of an equivalent dose. Both designs can shorten the trial duration and reduce the number of patients included by allowing for rapid dose escalation, potentially in single patient cohorts, in the absence of any DLT's. As such, they more rapidly approximate the MTD and reduce the number of patients treated at subtherapeutic dose levels (Van Brummelen et al. J Pharmacokinet Pharmacodyn. 2016). Alternatively, the predicted equivalent ritonavir-boosted oral cabazitaxel dose may be used in the design of a Bayesian model-based phase I study following the continual reassessment method (CRM) (O'Quigley et al. Biometrics. 1990) or escalation with overdose control (EWOC) design (Babb et al. Stat Med. 1998) .

Regardless of the chosen design, a first-in-human (FIH) study may take the following considerations into account: 1) The selection of an appropriate first in human dose may be guided by the predicted boosted oral dose of 7.4 mg is expected to approximate the MTD. Commonly, a 10-fold safety margin based on the STD 10 in animals is used in the selection of a starting dose (International Council for Harmonisation of Technical Requirements for Pharmaceuticals for Human Use. ICH S9 - Non-Clinical evaluation for anti-Cancer pharmaceuticals. Gl. 2013). As stated above, the Ki estimate, and as a result the predicted equivalent dose levels, are likely rather conservative. Given the well-informed approach and the conservative prediction of an equivalent dose, a more lenient and practical ~5-fold safety margin resulting in a proposed FIH dose of 1.5 mg may also be considered. 2) The dose levels to be investigated. Given the conservative predicted equivalent doses, it might be worthwhile to select a range of doses around, instead of only below the predicted MTD. In addition, practical restrictions in the selection of dose levels may exist depending on the drug content of tablets. 3) Lastly, in the definition of cohort sizes, it may be tempting to allow for small or even single patient cohorts to enable rapid dose escalation in the absence of toxicity. It is, however, important to consider the high interpatient variability in cabazitaxel PK, which should warrant caution for implementation of very small cohort sizes in the escalation phase of the design. Ultimately, the results of the Examples may be used to inform phase I study design, leading to a reduction in costs, the number of patients treated at subtherapeutic dose levels, and potentially the total number of patients required in phase I studies.

Whilst the present invention has been described and illustrated with reference to particular embodiments, it will be appreciated by those of ordinary skill in the art that the invention lends itself to many different variations not specifically illustrated herein. By way of example only, certain possible variations will now be described.

**Claims**

1. A pharmaceutical composition comprising an effective amount of cabazitaxel for use in a combination treatment in the treatment of cancer in a patient, wherein:

    the combination treatment further comprises a CYP3A inhibitor; and
    the dose of the CYP3A inhibitor is equivalent to 40 to 120 mg ritonavir.

2. A CYP3A inhibitor for use in a combination treatment in the treatment of cancer in a patient;
    wherein:
    the combination treatment further comprises a pharmaceutical composition comprising an effective amount of cabazitaxel; and wherein:
    the dose of the CYP3A inhibitor is equivalent to 40 to 120 mg ritonavir .

3. The pharmaceutical composition for use in accordance with claim 1, or the CYP3A inhibitor for use in accordance with claim 2, wherein cabazitaxel is formulated as an oral formulation, an intravenous formulation, or a suppository formulation; and/or wherein the CYP3A inhibitor is ritonavir.

4. The pharmaceutical composition or the CYP3A inhibitor for use in accordance with any of claims 1 to 3, wherein the pharmaceutical composition and the CYP3A inhibitor are formulated as a mixed formulation, or formulated in separate compositions; and/or wherein the pharmaceutical composition and the CYP3A inhibitor are provided simultaneously or sequentially within a time frame of about 4 hours.

5. The pharmaceutical composition or the CYP3A inhibitor for use in accordance with any of claims 1 to 4, wherein the cabazitaxel exposure level as measured in area under curve ($AUC_{0-inf}$) is within the range of 500 to 5000 ng.h/mL.

6. The pharmaceutical composition or the CYP3A inhibitor for use in accordance with any of claims 1 to 5, wherein said cancer is a solid tumor, for example a solid tumor selected from the group consisting of non-small cell lung cancer, breast cancer, gastric cancer, head and neck cancer, and prostate cancer; and, preferably, the said cancer is metastatic castration-resistant prostate cancer (mCRPC).

7. The pharmaceutical composition or the CYP3A inhibitor for use of any in accordance with claims 1 to 6, wherein:

    a) cabazitaxel is administered orally once every three weeks, and, optionally, at a dosage of 2.5 to 15 mg per administration; or:
    b) Cabazitaxel is administered orally once every week, and, optionally, at a dosage of 1 to 5 mg per administration.

8. The pharmaceutical composition or the CYP3A inhibitor for use in accordance with any of claims 1 to 7, wherein the CYP3A inhibitor is provided between 10 to 60 minutes, preferably between 10 to 30 minutes, prior to the provision of the pharmaceutical composition; and/or wherein the patient has developed resistance against first line therapy, for example docetaxel treatment, prior to the provision of the combination treatment.

9. A method for the treatment of a cancer in a patient, comprising:

    providing a pharmaceutical composition comprising an effective amount of cabazitaxel; and,
    providing a CYP3A inhibitor, wherein the dose of the CYP3A inhibitor is equivalent to 40 to 120 mg ritonavir.

10. The method for the treatment of a cancer in accordance with claim 9, wherein the method further comprises, prior to the provision of the oral formulation of cabizitaxel and/or the CYP3A inhibitor, treating the patient with a first line therapy, for example docetaxel; and/or wherein the cancer is a metastatic castration-resistant prostate cancer (mCRPC); and/or wherein the CYP3A inhibitor is provided from 10 to 60 minutes, preferably from 10 to 30 minutes, prior to the provision of the pharmaceutical composition; and/or the CYP3A inhibitor is ritonavir.

11. A method for the treatment of a cancer in a patient, comprising the steps of:

   - determining the activity of CYP3A in the patient;
   - optionally, comparing the activity of CYP3A to a reference level;
   - determining a CYP3A inhibitor daily dosage within the range equivalent to 40 to 120 mg ritonavir, based on the activity level of CYP3A determined in the patient.

12. A kit comprising a pharmaceutical composition comprising an effective amount of cabazitaxel and a CYP3A inhibitor in a dose equivalent to 40 to 120 mg ritonavir.

13. The kit according to claim 12, wherein the pharmaceutical composition comprising an effective amount of cabazitaxel is an oral composition, and/or wherein the CYP3A inhibitor is ritonavir; and/or wherein said kit is for the treatment of a solid tumor, in particular non-small cell lung cancer, gastric cancer, a breast cancer, a head and neck cancer or a prostate cancer, more in particular mCRPC.

14. A kit comprising a pharmaceutical composition comprising an effective amount of cabazitaxel and a pharmaceutical composition comprising a CYP3A inhibitor, wherein said kit is for use in a combination treatment as defined in any one of claims 1 to 7.

15. The pharmaceutical composition or the CYP3A inhibitor for use in accordance with any of claims 1 to 7, wherein the combination treatment further comprises the use of one or more anti-cancer agent, and/or the use of radiotherapy, and/or the use of immunotherapy.

Figure 1

Figure 2

Figure 3

Figure 4

Figure 5

Figure 6

Figure 7

A

B

Figure 8

Figure 9

Figure 10

A

- ● Wild-type - Vehicle
- ■ Wild-type - Vehicle follow-up
- Wild-type - RTV 1 mg/kg
- Wild-type - RTV 10 mg/kg
- ● FVB/NRj - RTV 25 mg/kg
- Cyp3aXAV - Vehicle
- Cyp3aXAV - Vehicle follow-up
- Cyp3aXAV - RTV 1 mg/kg
- Cyp3aXAV - RTV 10 mg/kg
- Cyp3aXAV - RTV 25 mg/kg

B

Figure 11

Figure 12

Figure 13

Figure 14

Figure 15

Figure 16

Figure 17

Figure 18

Figure 19

Figure 20

Figure 21

A

B

Figure 22

(●) represent concentration time profiles of Cabazitaxel co-administered with vehicle solution. (△) representative concentration time profiles of oral Cabazitaxel administered concomitant with a dose of 1 mg/kg ritonavir, (◇)10 mg/kg ritonavir, and (□)25 mg/kg ritonavir. The dashed line represents the LLOQ of 10 ng/mL.

Figure 23

(△) representative concentration time profiles of oral Cabazitaxel administered concomitant with a dose of 1 mg/kg ritonavir, (◇)10 mg/kg ritonavir, and (□)25 mg/kg ritonavir. The dotted line represents the LLOQ of 50ng/mL applicable to the samples measured in study A and B. The dashed line represents the LLOQ of 5 ng/mL applicable to the samples measured in study C.

Figure 24

$$Cl_{int,\ t} = \frac{CL_{int}}{1 + \frac{[RTV]_{central})}{K_i}}$$

Figure 25

Observed versus population predictions (A), Observed versus individual predictions (B), Conditional weighted residuals versus population predictions (C) and conditional weighted residuals versus time (D). (△)1 mg/kg ritonavir, (◇)10 mg/kg ritonavir, and (□)25 mg/kg ritonavir

Figure 26

Figure 27

Observed versus population predictions (A), Observed versus individual predictions (B), Conditional weighted residuals versus population predictions (C) and conditional weighted residuals versus time (D). (●) Cabazitaxel administered concomitant with vehicle solution, (△) concomitant with a dose of 1 mg/kg ritonavir, (◇) concomitant with a dose of 10 mg/kg ritonavir, and (□) concomitant with a dose of 25 mg/kg ritonavir

Figure 28

(A) Cabazitaxel without boosting, (B) cabazitaxel concomitant with 1 mg/kg ritonavir, (C) 10 mg/kg ritonavir, and (D) 25 mg/kg ritonavir. The solid and dashed lines represent the 50th, 5th and 95th percentiles of the observations. The shaded areas are the 95% confidence intervals for the 5th, 50th, and 95th percentiles.

Figure 29

Figure 30

$$Cl_{int,\ t} = \frac{CL_{int}}{1 + \frac{[RTV]_{central})}{K_i}}$$

Figure 31

Europäisches
Patentamt
European
Patent Office
Office européen
des brevets

# EUROPEAN SEARCH REPORT

Application Number

EP 22 21 3139

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TANG SENG CHUAN ET AL: "P-glycoprotein, CYP3A, and Plasma Carboxylesterase Determine Brain Disposition and Oral Availability of the Novel Taxane Cabazitaxel (Jevtana) in Mice", MOLECULAR PHARMACEUTICS, vol. 12, no. 10, 15 September 2015 (2015-09-15), pages 3714-3723, XP093044464, US ISSN: 1543-8384, DOI: 10.1021/acs.molpharmaceut.5b00470 Retrieved from the Internet: URL:https://pubs.acs.org/doi/pdf/10.1021/a cs.molpharmaceut.5b00470> * page 3715, 1-col page 3720 page 3721 * | 1-15 | INV. A61K31/337 A61K31/427 A61P35/00 |
| X | VAN DER PUTTEN E ET AL: "Reversal of resistance to docetaxel and cabazitaxel in prostate cancer cells with ritonavir", EUROPEAN JOURNAL OF CANCER, ELSEVIER, AMSTERDAM NL, vol. 174, 1 October 2022 (2022-10-01), XP087219769, ISSN: 0959-8049, DOI: 10.1016/S0959-8049(22)01090-5 [retrieved on 2022-10-28] * the whole document * | 1-15 | TECHNICAL FIELDS SEARCHED (IPC) A61K A61P |

-/--

The present search report has been drawn up for all claims

| Place of search | Date of completion of the search | Examiner |
|---|---|---|
| Munich | 4 May 2023 | Albayrak, Timur |

Europäisches Patentamt
European Patent Office
Office européen des brevets

# EUROPEAN SEARCH REPORT

Application Number

EP 22 21 3139

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JEROEN J M A HENDRIKX ET AL: "P-glycoprotein and cytochrome P450 3A act together in restricting the oral bioavailability of paclitaxel", INTERNATIONAL JOURNAL OF CANCER, JOHN WILEY & SONS, INC, US, vol. 132, no. 10, 14 November 2012 (2012-11-14), pages 2439-2447, XP071287021, ISSN: 0020-7136, DOI: 10.1002/IJC.27912 * the whole document * | 1-15 | |

TECHNICAL FIELDS SEARCHED (IPC)

The present search report has been drawn up for all claims

| Place of search | Date of completion of the search | Examiner |
|---|---|---|
| Munich | 4 May 2023 | Albayrak, Timur |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.............................................................................................

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009027644 A
- WO 2010020799 A

### Non-patent literature cited in the description

- **PETRYLAK et al.** *N Engl J Med.*, 2004
- **SERUGA et al.** *Nat Rev Clin Oncol.*, 2011
- **IKEZOE et al.** *Cancer Res.*, 2004
- **DE BONO et al.** *Lancet*, 2010
- **VRIGNAUD et al.** *Clin Cancer Res.*, 2013
- **SARANTOPOULOS et al.** *Cancer Chemother Pharmacol.*, 2014
- **MOES et al.** *Drug Deliv. Transl. Res.*, 2013
- **ER-JIAWANG et al.** *Chem. Res. Toxicol.*, 2001
- **WACHER et al.** *Mol Carc.*, 1995
- **HENDRIKX et al.** *J. Chrom. B.*, 2011
- **HENDRIKX et al.** *Int J Cancer.*, 2015
- **VAN WATERSCHOOT et al.** *Cancer Res.*, 2009
- **BEAL SL SL.** *NONMEM user guides*, 1989
- **LINDBOM et al.** *Comput Methods Programs Biomed.*, 2005
- **ZHANG et al.** *J Pharmacokinet Pharmacodyn.*, 2003
- **YU et al.** *J Clin Pharmacol.*, 2020
- **WILKINSON et al.** *Clin Pharmacol Ther.*, 1975
- **DOSNE et al.** *J Pharmacokinet Pharmacodyn.*, 2017
- **HSU et al.** *Clin Pharmacokinet.*, 1998
- **HENDRIKX et al.** *Int J Cancer.*, 2013
- **HUISMAN et al.** *J Pharmacol Exp Ther.*, 2003
- **FERRON et al.** *Cancer Chemother Pharmacol.*, 2013
- **LEEN et al.** *Gut.*, 1992
- **KAN et al.** *J Nucl Med.*, 1979
- **DAMOISEAUX et al.** *Pharmaceuticals*, 2022
- **KOOLEN et al.** *J Clin Pharmacol.*, 2012
- **KAPPELHOFF et al.** *Br J Clin Pharmacol.*, 2005
- **MITA et al.** *Clin Cancer Res.*, 2009

- **DIÉRAS et al.** *Eur J Cancer.*, 2013
- **JOERGER et al.** *Clin Cancer Res.*, 2007
- **LIN et al.** *Drug Metab Dispos.*, 2013
- **JIBODH et al.** *Eur J Pharmacol.*, 2013
- **SAWICKI et al.** *Int J Pharm.*, 2016
- **VERMUNT et al.** *Cancer Rep (Hoboken)*, 2021
- **TANG et al.** *Mol Pharm.*, 2015
- **ROCK et al.** *Mol Pharmacol.*, 2014
- **DARWICH et al.** *Drug Metab Dispos.*, 2014
- **KHARASCH et al.** *Clin Pharmacol Ther.*, 2008
- **SZAKÁCS et al.** *Drug Discov Today.*, 2008
- **CHOUDHURI et al.** *Int J Toxicol.*, 2006
- **HOLMSTOCK et al.** *Drug Metab Dispos.*, 2010
- **BACHMEIER et al.** *Pharm Res.*, 2005
- **DE WEGER et al.** *Clin Pharmacol Drug Dev.*, 2021
- **MCBEAN et al.** *J Clin Imaging Sci.*, 2021
- **BHAMBHVANI et al.** *World Neurosurg.*, 2020
- **MYINT et al.** *Med Sci Monit.*, 2021
- **NAIR et al.** *J Basic Clin Pharm.*, 2016
- **EICHBAUM et al.** *Eur J Clin Pharmacol.*, 2013
- **KOOLEN et al.** *Br J Clin Pharmacol.*, 2010
- **DE VRIES SCHULTINK et al.** *Cancer Med.*, 2019
- **ABBVIE BV. NORVIR.** *INN-ritonavir-Summary of Product Characteristics*, 2019
- **MOES et al.** *Eur J Pharm Biopharm.*, 2013
- **MOES et al.** *Int J Pharm.*, 2011
- **LOOS et al.** *Clin Pharmacol Ther.*, 2003
- **LE TOURNEAU et al.** *J Natl Cancer Inst.*, 2009
- **SIMON et al.** *J Natl Cancer Inst.*, 1997
- **YUAN et al.** *Clin Cancer Res.*, 2016
- **VAN BRUMMELEN et al.** *J Pharmacokinet Pharmacodyn.*, 2016
- **O'QUIGLEY et al.** *Biometrics*, 1990
- **BABB et al.** *Stat Med.*, 1998